(19) Europäisches Patentamt
European Patent Office
Office européen des brevets

(11) EP 3 753 968 A1

(12) **EUROPEAN PATENT APPLICATION**
published in accordance with Art. 153(4) EPC

(43) Date of publication:
**23.12.2020 Bulletin 2020/52**

(21) Application number: **19755163.3**

(22) Date of filing: **24.01.2019**

(51) Int Cl.:
***C08G 65/329*** *(2006.01)* ***C08G 65/26*** *(2006.01)*
***C11D 1/72*** *(2006.01)*

(86) International application number:
**PCT/JP2019/002321**

(87) International publication number:
**WO 2019/159643 (22.08.2019 Gazette 2019/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.02.2018 JP 2018024125**
**05.12.2018 JP 2018228092**

(71) Applicant: **Nippon Shokubai Co., Ltd.**
**Osaka-shi, Osaka 541-0043 (JP)**

(72) Inventors:
- **MICHITAKA, Daisuke**
  **Suita-shi Osaka 5640034 (JP)**
- **NISHIKAWA, Tsuyoshi**
  **Suita-shi Osaka 5640034 (JP)**

(74) Representative: **Cabinet Beau de Loménie**
**158, rue de l'Université**
**75340 Paris Cedex 07 (FR)**

# (54) POLYALKYLENE GLYCOL-CONTAINING COMPOUND

(57) The present invention provides a polyalkylene glycol-containing compound having higher detergency than conventional polyalkylene glycol-containing compounds. The present invention relates to a polyalkylene glycol-containing compound represented by the following formula (1-1) .

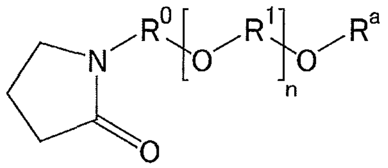

(1-1)

EP 3 753 968 A1

## Description

### TECHNICAL FIELD

The present invention relates to polyalkylene glycol-containing compounds. Specifically, the present invention relates to polyalkylene glycol-containing compounds useful for detergents, for example.

### BACKGROUND ART

Polyalkylene glycol chain-containing compounds with appropriately adjusted chain lengths and alkylene oxides that constitute the compounds have properties such as hydrophilicity, hydrophobicity, flexibility, and steric repulsion. Such compounds are used in various applications such as detergent builders, detergents, chemicals for water treatment, scale inhibitors, and dispersants.

For example, Patent Literature 1 discloses a polyalkylene glycol represented by a specific structure for the use as a scale inhibitor, a detergent composition, or a dispersant for pigments.

### CITATION LIST

- Patent Literature

Patent Literature 1: JP 2012-149186 A

### SUMMARY OF INVENTION

- Technical Problem

Although the polyalkylene glycol represented by a specific structure for a detergent composition is disclosed in Patent Literature 1 as described above, conventional polyalkylene glycol-containing compounds still need to be improved in terms of detergency.

The present invention has been made in view of such a current state of the art and aims to provide a polyalkylene glycol-containing compound having higher detergency than conventional polyalkylene glycol-containing compounds.

- Solution to Problem

The present inventors have conducted various studies on polyalkylene glycol-containing compounds and found that a polyalkylene glycol-containing compound having a specific structure with a hydrophobic group-terminated polyalkylene glycol chain has higher detergency than conventional polyalkylene glycol-containing compounds. Thereby, the inventors have arrived at the solution to the above problem, completing the present invention.

That is, a first aspect of the present invention relates to a polyalkylene glycol-containing compound represented by the following formula (1-1):

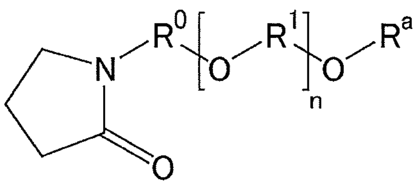

(1-1)

wherein $R^0$ is a C1-C20 divalent organic group; $R^1$s are the same as or different from each other and are each a C2-C20 alkylene group; $R^a$ is an optionally substituted C1-C20 alkyl group, an optionally substituted C6-C20 aryl group, or an optionally substituted C1-C20 heterocyclic group; and n is an average number of moles of oxyalkylene groups added and is 1 to 300.

Preferably, $R^a$ is a C1-C20 alkyl group or a group represented by the following formula (1-2):

$$-CH_2CH(OR^{a2})CH_2-OR^{a1} \qquad (1-2)$$

wherein $R^{a1}$ is a C1-C17 alkyl or heterocyclic group, or a C6-C17 aryl group; and $R^{a2}$ is a hydrogen atom, an optionally substituted C1-C16 alkyl group, an optionally substituted C6-C16 aryl group, or an optionally substituted C1-C16 hete-

rocyclic group.

Preferably, $R^0$ is a C1-C20 alkylene group; a group represented by the following formula (1-4):

$$-(CH_2)_p-O-CH_2CH(OR^{01})CH_2- \quad (1\text{-}4)$$

wherein $R^{01}$ is a hydrogen atom or a C1-C17 organic group and p is 0 to 10; a group represented by the following formula (1-7):

$$-(CH_2)_{p'}-CH(OH)CH_2- \quad (1\text{-}7)$$

wherein p' is 0 to 5; or a group represented by the following formula (1-8):

$$-R^{03}-S-R^{04}- \quad (1\text{-}8)$$

wherein $R^{03}$ and $R^{04}$ are the same as or different from each other and are each an optionally substituted C1-C10 alkylene group.

A second aspect of the present invention relates to a polyalkylene glycol-containing compound represented by the following formula (2-1):

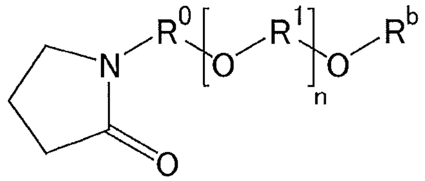

(2-1)

wherein $R^0$ is a C1-C20 divalent organic group; $R^1$s are the same as or different from each other and are each a C2-C20 alkylene group; $R^b$ is a C21-C50 organic group; and n is an average number of moles of oxyalkylene groups added and is 1 to 300.

Preferably, $R^b$ is a group represented by the following formula (2-2):

$$-CH_2CH(OR^{b2})CH_2OR^{b1} \quad (2\text{-}2)$$

wherein $R^{b1}$ is a C1-C47 organic group; and $R^{b2}$ is a hydrogen atom or a C1-C46 organic group.

Preferably, $R^b$ is an optionally substituted C21-C30 alkyl group or an optionally substituted C21-C30 aryl group.

Preferably, $R^0$ is a C1-C20 alkylene group; a group represented by the following formula (1-4):

$$-(CH_2)_p-O-CH_2CH(OR^{01})CH_2- \quad (1\text{-}4)$$

wherein $R^{01}$ is a hydrogen atom or a C1-C17 organic group and p is 0 to 10; a group represented by the following formula (1-7):

$$-(CH_2)_{p'}-CH(OH)CH_2- \quad (1\text{-}7)$$

wherein p' is 0 to 5; or a group represented by the following formula (1-8):

$$-R^{03}-S-R^{04}- \quad (1\text{-}8)$$

wherein $R^{03}$ and $R^{04}$ are the same as or different from each other and are each an optionally substituted C1-C10 alkylene group.

A third aspect of the present invention relates to a polyalkylene glycol-containing compound represented by the following formula (3-1):

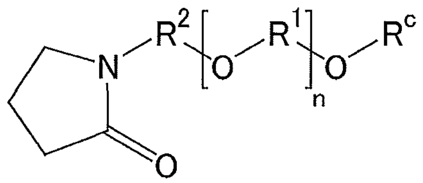

(3-1)

wherein $R^2$ is a C21-C100 divalent organic group; $R^1$s are the same as or different from each other and are each a C2-C20 alkylene group; $R^c$ is an optionally substituted C1-C50 alkyl group, an optionally substituted C6-C50 aryl group, or an optionally substituted C1-C50 heterocyclic group; and n is an average number of moles of oxyalkylene groups added and is 1 to 300.

Preferably, $R^2$ is a group represented by the following formula (3-5):

$$-CH_2CH_2OCH_2CH(OR^{22})CH_2- \qquad (3\text{-}5)$$

wherein $R^{22}$ is a C16-C95 organic group.

The present invention also relates to a composition including:

the polyalkylene glycol-containing compound; and
a different component other than the compound,
the polyalkylene glycol-containing compound being present in an amount of 1 to 99% by mass based on 100% by mass of the composition.

The present invention also relates to a detergent composition including:
the polyalkylene glycol-containing compound; and a detergent additive other than the compound.

- Advantageous Effects of Invention

The polyalkylene glycol-containing compounds of the present invention having the above-described features have higher detergency than conventional polyalkylene glycol-containing compounds, and are thus suitable for detergents, for example.

DESCRIPTION OF EMBODIMENTS

The following description is offered to specifically illustrate preferred embodiments of the present invention. It should be noted that the present invention is not limited only to these embodiments, and the embodiments can be appropriately altered within the scope of the present invention. Any combination of two or more of the following preferred embodiments of the present invention is also a preferred embodiment of the present invention.

The matters referring simply to "the present invention" herein are common to the first to third aspects of the present invention.

<First aspect of the present invention>

A polyalkylene glycol-containing compound according to the first aspect of the present invention (hereinafter, also referred to as a PAG compound according to the first aspect of the present invention) is represented by the formula (1-1).

In the formula (1-1), $R^a$ is an optionally substituted C1-C20 alkyl group, an optionally substituted C6-C20 aryl group, or an optionally substituted C1-C20 heterocyclic group.

The PAG compound according to the first aspect of the present invention includes a pyrrolidone structure having an interaction with a carboxyl group or an ester group in stains such as sebum and at an end of the polyalkylene glycol chain a hydrophobic group represented by $R^a$ with a specific structure. This PAG compound has a strong interaction with stains (in particular, oily stains) and can reduce the interfacial tension between water and oil, leading to improvement of detergency. The PAG compound according to the first aspect of the present invention having the aforementioned structure has a good balance between hydrophobicity and hydrophilicity, and thus has good detergency and anti-redeposition ability to composite stains of hydrophilic stains and hydrophobic stains.

The number of carbon atoms of the alkyl group is preferably 2 to 19, more preferably 3 to 18, still more preferably 4 to 17, further preferably 6 to 16, still further more preferably 8 to 15, particularly preferably 12 to 14.

The number of carbon atoms of the alkyl group includes the number of carbon atoms of a substituent.

The number of carbon atoms of each of the below-described optionally substituted hydrocarbon groups also includes the number of carbon atoms of a substituent.

Examples of the alkyl group include aliphatic alkyl groups such as methyl, ethyl, n-propyl, n-butyl, n-pentyl (amyl), n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, i-propyl, sec-butyl, i-butyl, t-butyl, 1-methylbutyl, 1-ethylpropyl, 2-methylbutyl, i-amyl, neopentyl, 1,2-dimethylpropyl, 1,1-dimethylpropyl, t-amyl, 1,3-dimethylbutyl, 3,3-dimethylbutyl, 2-ethylbutyl, 2-ethyl-2-methylpropyl, 1-methylheptyl, 2-ethylhexyl, 1,5-dimethylhexyl, t-octyl, branched nonyl, branched decyl, branched undecyl, branched dodecyl, branched tridecyl, branched tetradecyl, branched pentadecyl, branched hexadecyl, branched heptadecyl, branched stearyl, and branched icosyl groups; and alicyclic alkyl groups such as cyclopropyl, cyclopropylmethyl, cyclobutyl, cyclobutylmethyl, cyclopentyl, cyclohexyl, cyclohexylmethyl, cycloheptyl, cyclooctyl, cyclohexylpropyl, cyclododecyl, norbornyl (C7), adamantyl (C10), and cyclopentylethyl groups. The alkyl group is preferably an aliphatic alkyl group.

The number of carbon atoms of the aryl group is preferably 6 to 19, more preferably 6 to 16, still more preferably 6 to 10, particularly preferably 6 to 8.

Examples of the aryl group include phenyl, benzyl, phenethyl, o-, m-, or p-tolyl, 2,3- or 2,4-xylyl, mesityl, naphthyl, anthryl, phenanthryl, biphenylyl, benzhydryl, and trityl groups and a group in which any of the above alkyl groups bonds to an aromatic ring such as a phenyl group or a naphthyl group. The aryl group is preferably a phenyl group or a benzyl group.

The number of carbon atoms of the heterocyclic group is preferably 1 to 19, more preferably 2 to 16, still more preferably 2 to 10, particularly preferably 2 to 8.

The heterocyclic group is preferably an aromatic heterocyclic group having aromaticity, and examples include pyrrole, furan, thiophene, imidazole, pyrazole, oxazole, isooxazole, thiazole, isothiazole, tetrazole, pyridinyl, pyrimidinyl, pyridazinyl, pyrazinyl, 1,2,3-triazinyl, quinolinyl, isoquinolinyl, quinazolinyl, phthalazinyl, buteridinyl, coumarinyl, chromonyl, 1,4-benzodiazepinyl, indole, benzimidazole, benzofuranyl, purinyl, acridinyl, phenoxazinyl, and phenothiazinyl groups and a group in which any of the above alkyl groups bonds to an aromatic heterocycle. Preferred among these are a tetrazole group and an imidazole group.

Examples of a substituent of each of the optionally substituted alkyl, aryl, and heterocyclic groups include hydroxy, alkoxy, carboxyl, acyl, sulfonic acid, amino, phosphoric acid, ether, thioether, carbonyl, and ester groups. Preferred are a hydroxy group and an alkoxy group.

When $R^a$ contains a substituent, $R^a$ is preferably a group represented by the following formula (1-2):

$$-CH_2CH(OR^{a2})CH_2-OR^{a1} \qquad (1\text{-}2)$$

wherein $R^{a1}$ is a C1-C17 alkyl or heterocyclic group, or a C6-C17 aryl group; and $R^{a2}$ is a hydrogen atom, an optionally substituted C1-C16 alkyl group, an optionally substituted C6-C16 aryl group, or an optionally substituted C1-C16 heterocyclic group.

For example, in a second step of the below-described method (i) of producing the PAG compound according to the first aspect of the present invention, specifically in reacting a reaction product obtained in a first step with a hydrophobic group-containing compound containing a glycidyl ether group, a PAG compound in which $R^a$ is a group represented by the formula (1-2) is produced.

Specific examples of the alkyl, aryl, and heterocyclic groups for $R^{a1}$ are included in those exemplified above.

The number of carbon atoms of the alkyl group for $R^{a1}$ is preferably 8 to 14, more preferably 12 to 14.

The number of carbon atoms of the aryl group for $R^{a1}$ is preferably 6 to 14, more preferably 6 to 12, still more preferably 6 to 10, particularly preferably 6 to 8.

The number of carbon atoms of the heterocyclic group for $R^{a1}$ is preferably 2 to 14, more preferably 2 to 12, still more preferably 2 to 8.

When $R^{a2}$ is an optionally substituted C1-C16 alkyl group, an optionally substituted C6-C16 aryl group, or an optionally substituted C1-C16 heterocyclic group, $R^{a2}$ is preferably a group represented by the following formula (1-3) :

$$-[CH_2CH(CH_2-O-R^{a3})-O]_m-H \qquad (1\text{-}3)$$

wherein $R^{a3}$s are the same as or different from each other and are each a C1-C13 alkyl group, a C6-C13 aryl group, or a C1-C13 heterocyclic group; and m is a number of 1 to 4.

For example, in a second step of the below-described method (i) of producing the PAG compound according to the first aspect of the present invention, specifically in reacting a reaction product obtained in a first step with a hydrophobic group-containing compound containing a glycidyl ether group, a PAG compound with m in the formula (1-3) being 1 is produced by reacting the hydroxy group of-$CH_2CH(OH)CH_2-OR^{a1}$ obtained with one molecule of a hydrophobic group-containing compound containing a glycidyl ether group. Further, a PAG compound with m being 2 is produced

by reacting the hydroxy group of the resulting PAG compound with one molecule of a hydrophobic group-containing compound containing a glycidyl ether group.

Specific examples of the alkyl, aryl, and heterocyclic groups for $R^{a3}$ are included in those exemplified above.

The number of carbon atoms of the alkyl group for $R^{a3}$ is preferably 4 to 13, more preferably 8 to 12.

The number of carbon atoms of the aryl group for $R^{a3}$ is preferably 6 to 13, more preferably 6 to 10.

The number of carbon atoms of the heterocyclic group for $R^{a3}$ is preferably 1 to 13, more preferably 2 to 10.

$R^{a3}$ is preferably a C1-C13 alkyl group. Alternatively, $R^{a3}$ is preferably the same group as $R^{a1}$.

Preferably, m is 1.

In the formula (1-1), $R^a$ is preferably a C1-C20 alkyl group or a group represented by the formula (1-2) with $R^{a2}$ being a hydrogen atom, more preferably a C1-C20 alkyl group, still more preferably a linear or branched unsubstituted aliphatic alkyl group.

In the formula (1-1), $R^1$s "are the same as or different from each other" and are each a C2-C20 alkylene group. This means that all n alkylene groups for $R^1$ in the polyalkylene glycol may be the same as or different from each other.

In the formula (1-1), the oxyalkylene group represented by $R^1O$ is an alkylene oxide adduct. Examples of the alkylene oxide include C2-C8 alkylene oxides such as ethylene oxide, propylene oxide, butylene oxide, isobutylene oxide, 1-butene oxide, 2-butene oxide, and styrene oxide. Preferred are C2-C4 alkylene oxides such as ethylene oxide, propylene oxide, and butylene oxide, with ethylene oxide and propylene oxide being more preferred.

When the polyalkylene glycol is an adduct with any two or more alkylene oxides selected from alkylene oxides such as ethylene oxide, propylene oxide, butylene oxide, and styrene oxide, the alkylene oxides may be added by any addition form such as random addition, block addition, or alternating addition. To balance hydrophilicity and hydrophobicity, the oxyalkylene groups in the polyalkylene glycol preferably essentially include oxyethylene groups. More preferably, the oxyethylene groups constitute 50 mol% or more of the oxyalkylene groups, still more preferably constitute 90 mol% or more of the oxyalkylene groups.

In the formula (1-1), n is the average number of moles of the oxyalkylene groups added, and n is 1 to 300, preferably 2 to 200, more preferably 3 to 100, still more preferably 5 to 50, particularly preferably 7 to 30.

In the formula (1-1), $R^0$ is a C1-C20 divalent organic group.

The number of carbon atoms of the divalent organic group is preferably 2 to 15, more preferably 2 to 10, still more preferably 2 to 8, particularly preferably 2 to 5.

The divalent organic group may be any group and is preferably an optionally substituted divalent hydrocarbon group.

Examples of the divalent hydrocarbon group include a group obtained by abstracting one hydrogen atom from any of monovalent hydrocarbon groups such as the aliphatic alkyl groups, the alicyclic alkyl groups, and the aryl groups exemplified for $R^a$.

Specific examples of a substituent of the optionally substituted divalent hydrocarbon group are as described above, and preferred are hydroxy, alkoxy, ether, thioether, and carbonyl groups.

The divalent hydrocarbon group containing such a preferred substituent is preferably a group represented by any of the formulas (1-4), (1-7), and (1-8) described below.

Preferably, the divalent hydrocarbon group is an alkylene group obtained by abstracting one hydrogen atom from an aliphatic alkyl group or an alicyclic alkyl group.

In other words, $R^0$ is preferably a C1-C20 alkylene group.

Examples of the alkylene group include methylene, methylmethylene, ethylene, n-propylene, isopropylene, n-butylene, isobutylene, sec-butylene, tert-butylene, n-pentylene, isopentylene, neopentylene, hexylene, heptylene, octylene, nonylene, decylene, undecylene, dodecylene, tridecylene, tetradecylene, pentadecylene, hexadecylene, heptadecylene, and octadecylene groups.

When the divalent hydrocarbon group contains a substituent, $R^0$ is preferably a group represented by the following formula (1-4):

$$-(CH_2)_p-O-CH_2CH(OR^{01})CH_2- \qquad (1\text{-}4)$$

wherein $R^{01}$ is a hydrogen atom or a C1-C17 organic group; and p is a number of 0 to 10.

Preferably, p is a number of 1 to 8, more preferably a number of 2 to 5.

The organic group for $R^{01}$ may be an optionally substituted hydrocarbon group.

The hydrocarbon group may be any of the above-described aliphatic alkyl, alicyclic alkyl, and aryl groups. Specific examples of a substituent of the optionally substituted hydrocarbon group are as described above.

The C1-C17 organic group is preferably a group represented by the following formula (1-5):

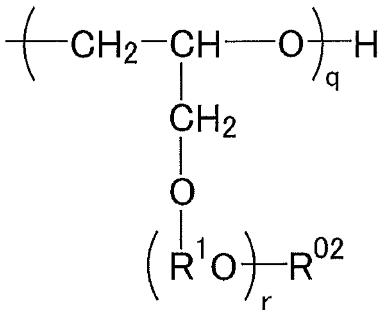

(1-5)

wherein $R^{02}$ is the same as or different from each other and is a C1-C12 alkyl group; q is 1 or 2; $R^1$s are the same as or different from each other and are each a C2-C20 alkylene group; and r is the average number of moles of oxyalkylene groups added and is 1 to 6.

$R^1$ in the formula (1-5) is the same group as $R^1$ in the formula (1).

Specific examples of the C1-C12 alkyl group for $R^{02}$ in the formula (1-5) are included in those exemplified for $R^a$.

The number of carbon atoms of the alkyl group for $R^{02}$ is preferably 4 to 12, more preferably 8 to 12. Alternatively, $R^{02}$ is preferably the same group as $R^a$.

Preferably, q is 1.

When $R^0$ is the group represented by the formula (1-4), $R^{01}$ is preferably a hydrogen atom.

The group represented by the formula (1-4) is preferably a group represented by the following formula (1-6) :

$-CH_2CH_2OCH_2CH(OH)CH_2-$ (1-6).

Preferably, $R^0$ is the group represented by the formula (1-6) or a C1-C20 alkylene group. The number of carbon atoms of the alkylene group is preferably 1 to 15, more preferably 2 to 10, still more preferably 2 to 8, particularly preferably 2 to 5.

In a preferred embodiment of the present invention, when the divalent hydrocarbon group contains a substituent, $R^0$ is a group represented by the following formula (1-7):

$-(CH_2)_{p'}-CH(OH)CH_2-$ (1-7)

wherein p' is 0 to 5.

Preferably, p' is 1 to 4, more preferably 1 to 3, most preferably 1.

In another preferred embodiment of the present invention, when the divalent hydrocarbon group contains a substituent, $R^0$ is a group represented by the following formula (1-8):

$-R^{03}-S-R^{04}-$ (1-8)

wherein $R^{03}$ and $R^{04}$ are the same as or different from each other and are each an optionally substituted C1-C10 alkylene group.

Specific examples of the C1-C10 alkylene group are as described above.

The number of carbon atoms of the alkylene group for $R^{03}$ is preferably 1 to 8, more preferably 1 to 6, still more preferably 1 to 4, most preferably 2.

The number of carbon atoms of the alkylene group for $R^{04}$ is preferably 1 to 8, more preferably 1 to 6, still more preferably 1 to 4, most preferably 3.

Specific examples of a substituent of the optionally substituted alkylene group are as described above.

$R^{03}$ is preferably an unsubstituted alkylene group, more preferably a methylene, methylmethylene, ethylene, n-propylene, isopropylene, n-butylene, isobutylene, sec-butylene, tert-butylene, n-pentylene, isopentylene, or neopentylene group, still more preferably a methylene, methylmethylene, ethylene, or n-propylene group, particularly preferably an ethylene group.

$R^{04}$ is preferably a C1-C5 alkylene group containing a substituent such as a carbonyl, hydroxy, carboxyl, or sulfonic acid group. $R^{04}$ is more preferably a carbonyl group-containing C1-C5 alkylene group. In the formula (1-1), the carbonyl carbon of the carbonyl group-containing alkylene group for $R^{04}$ preferably bonds to an oxygen atom derived from the polyalkylene glycol. Preferred alkylene groups for $R^{04}$ are the same as those for $R^{03}$.

The group represented by the formula (1-8) is preferably a group represented by the following formula (1-9) :

$-R^{05}-S-R^{06}CO-$ (1-9)

(wherein $R^{05}$ is a C1-C10 alkylene group; and $R^{06}$ is a C1-C9 alkylene group), still more preferably a group represented by the following formula (1-10):

$$-CH_2CH_2\text{-}S\text{-}CH_2CH_2\text{-}CO- \qquad (1\text{-}10).$$

The PAG compound according to the first aspect of the present invention preferably has a number average molecular weight of 500 to 100000, more preferably 600 to 50000, still more preferably 650 to 10000, particularly preferably 700 to 5000. The number average molecular weight of the PAG compound can be calculated based on the molecular weight and the number of each of the structural units of the PAG compound.

The proportion of a group represented by $R^a$ in the PAG compound according to the first aspect of the present invention represented by the formula (1-1) is preferably 2.5 to 60% by mass based on 100% by mass of the PAG compound. This can provide a PAG compound having more suitable hydrophobicity, which can lead to a higher interaction with stains such as sebum. The proportion of a group represented by $R^a$ is more preferably 3 to 50% by mass, still more preferably 4 to 40% by mass.

<Second aspect of the present invention>

A polyalkylene glycol-containing compound according to the second aspect of the present invention (hereinafter, also referred to as a PAG compound according to the second aspect of the present invention) is represented by the formula (2-1).

$R^0$, $R^1$, and n in the formula (2-1) are the same as $R^0$, $R^1$, and n in the formula (1-1).

$R^b$ in the formula (2-1) is a C21-C50 organic group.

The PAG compound according to the second aspect of the present invention also includes a pyrrolidone structure having an interaction with a carboxyl group or an ester group in stains such as sebum and at an end of the polyalkylene glycol chain an organic group represented by $R^b$ having a specific number of carbon atoms. This PAG compound has a strong interaction with stains and can reduce the interfacial tension between water and oil, leading to improvement of detergency.

Examples of the organic group include an optionally substituted hydrocarbon group and a hydrocarbon group optionally containing a heteroatom.

Examples of these hydrocarbon groups include aliphatic alkyl, alicyclic alkyl, alkenyl, alkynyl, and aryl groups.

Examples of the heteroatom-containing hydrocarbon group include the heterocyclic groups exemplified above.

Examples of the C21-C50 aliphatic alkyl group include henicosyl, docosyl, tricosyl, tetracosyl, pentacosyl, hexacosyl, heptacosyl, octacosyl, nonacosyl, triacontyl, pentatriacontyl, tetracontyl, pentatetracontyl, and pentacontyl groups.

Examples of the C21-C50 alicyclic alkyl group include alicyclic alkyl groups such as cyclopropyl octadecyl, cyclobutyl heptadecyl, cyclopentyl hexadecyl, cyclohexyl pentadecyl, cycloheptyl tetradecyl, and cyclooctyl tridecyl groups.

Examples of the C21-C50 alkenyl group include eicosenyl, henicosenyl, docosenyl, tricosenyl, tetracosenyl, pentacosenyl, hexacosenyl, heptacosenyl, octacosenyl, nonacosenyl, triacontenyl, pentatriacontenyl, tetracontenyl, pentatetracontenyl, and pentacontenyl groups.

Examples of the C21-C50 alkynyl group include eicosynyl, henicosynyl, docosynyl, tricosynyl, tetracosynyl, pentacosynyl, hexacosynyl, heptacosynyl, octacosynyl, nonacosynyl, triacontynyl, pentatriacontynyl, tetracontynyl, pentatetracontynyl, and pentacontynyl groups.

Examples of the C21-C50 aryl group include a quarterphenyl group and a quinquephenyl group and a group in which a group such as an alkyl group bonds to an aromatic ring of a quarterphenyl, quinquephenyl, phenyl, naphthyl, fluorenyl, anthracenyl, phenanthryl, biphenyl, terphenyl, or distyrenated phenyl group.

The number of carbon atoms of the C21-C50 organic group is preferably 21 to 40, more preferably 21 to 35, still more preferably 21 to 30, further preferably 21 to 28, particularly preferably 21 to 25.

In a preferred embodiment of the present invention, $R^b$ is an optionally substituted C21-C30 alkyl group or an optionally substituted C21-C30 aryl group.

When $R^b$ contains a substituent, $R^b$ is preferably a group represented by the following formula (2-2):

$$-CH_2CH(OR^{b2})CH_2OR^{b1} \qquad (2\text{-}2)$$

wherein $R^{b1}$ is a C1-C47 organic group; and $R^{b2}$ is a hydrogen atom or a C1-C46 organic group.

For example, in a second step of the below-described method (i) of producing the PAG compound according to the second aspect of the present invention, specifically in reacting a reaction product obtained in a first step with a

hydrophobic group-containing compound containing a glycidyl ether group, a PAG compound in which $R^b$ is a group represented by the formula (2-2) can be produced.

In the formula (2-2), $R^{b1}$ is a C1-C47 organic group and $R^{b2}$ is a hydrogen atom or a C1-C46 organic group. When $R^{b2}$ is a hydrogen atom, $R^{b1}$ is a C18-C47 organic group.

Examples of the organic group include hydrocarbon groups such as aliphatic alkyl, alicyclic alkyl, aryl, and heterocyclic groups.

Specific examples of the C1-C46 aliphatic alkyl, C1-C46 alicyclic alkyl, C1-C46 aryl, and C1-C46 heterocyclic groups are included in those exemplified above.

Specific examples of a substituent of the optionally substituted hydrocarbon group are as described above.

When $R^{b2}$ is a C1-C46 organic group, $R^{b2}$ is preferably a group represented by the following formula (2-3):

$$-[CH_2CH(CH_2\text{-}O\text{-}R^{b3})\text{-}O]_k\text{-}H \qquad (2\text{-}3)$$

wherein $R^{b3}$ are the same as or different from each other and are each a C1-C43 alkyl group; and k is 1 to 11.

For example, in a second step of the below-described method (i) of producing the PAG compound according to the second aspect of the present invention, specifically in reacting a reaction product obtained in a first step with a hydrophobic group-containing compound containing a glycidyl ether group, a PAG compound with k in the formula (2-3) being 1 is produced by reacting the hydroxy group of-$CH_2CH(OH)CH_2$-$OR^{b1}$ obtained with one molecule of a hydrophobic group-containing compound containing a glycidyl ether group. Further, a PAG compound with k being 2 is produced by reacting the hydroxy group of the resulting PAG compound with one molecule of a hydrophobic group-containing compound containing a glycidyl ether group.

Specific examples of the C1-C43 alkyl group for $R^{b3}$ are included in those exemplified above.

The number of carbon atoms of the alkyl group for $R^{b3}$ is preferably 1 to 40, more preferably 8 to 30.

Alternatively, $R^{b3}$ is preferably the same group as $R^{b1}$.

Preferably, k is 1.

In the formula (2-1), $R^b$ is preferably a C21-C50 alkyl group or a group represented by the formula (2-2) with $R^{b2}$ being a hydrogen atom.

The PAG compound according to the second aspect of the present invention preferably has a number average molecular weight of 500 to 100000, more preferably 600 to 50000, still more preferably 650 to 10000, particularly preferably 700 to 5000.

The proportion of a group represented by $R^b$ in the PAG compound according to the second aspect of the present invention represented by the formula (2-1) is preferably 2.5 to 60% by mass based on 100% by mass of the PAG compound. This can provide a PAG compound having more suitable hydrophobicity, which has a higher interaction with stains such as sebum. The proportion of a group represented by $R^b$ is more preferably 3 to 50% by mass, still more preferably 4 to 40% by mass.

<Third aspect of the present invention>

A polyalkylene glycol-containing compound according to the third aspect of the present invention (hereinafter, also referred to as a PAG compound according to the third aspect of the present invention) is represented by the formula (3-1).

$R^1$ and n in the formula (3-1) are the same as $R^1$ and n in the formula (1-1), respectively.

$R^2$ is a C21-C100 divalent organic group.

$R^c$ is an optionally substituted C1-C50 alkyl group, an optionally substituted C6-C50 aryl group, or an optionally substituted C1-C50 heterocyclic group.

The PAG compound according to the third aspect of the present invention includes a pyrrolidone structure having an interaction with a carboxyl group or an ester group in stains such as sebum, an organic group for $R^2$ having a specific number of carbon atoms between the pyrrolidone structure and the polyalkylene glycol chain, and at an end of the polyalkylene glycol chain a hydrophobic group represented by $R^c$ with a specific structure. This PAG compound has a strong interaction with stains and can reduce the interfacial tension between water and oil, leading to improvement of detergency.

Specific examples of the alkyl, aryl, and heterocyclic groups for $R^c$ are included in the above-described specific examples of the alkyl, aryl, and heterocyclic groups.

The number of carbon atoms of the optionally substituted alkyl group for $R^c$ is preferably 2 to 40, more preferably 4 to 30, still more preferably 8 to 20, particularly preferably 10 to 18, most preferably 12 to 14.

The number of carbon atoms of the optionally substituted aryl group for $R^c$ is preferably 6 to 40, more preferably 6 to 30, still more preferably 6 to 25.

The number of carbon atoms of the optionally substituted heterocyclic group for $R^c$ is preferably 1 to 40, more

preferably 2 to 30, still more preferably 2 to 20.

Specific examples of a substituent of each of the optionally substituted alkyl, aryl, and heterocyclic groups are as described above. Preferred are hydroxy, alkoxy, and ether groups.

When the alkyl, aryl, and heterocyclic groups for $R^c$ each have a substituent, $R^c$ is preferably a group represented by the following formula (3-2):

$-CH_2CH(OR^{c2})CH_2OR^{c1}$ (3-2)

wherein $R^{c1}$ is a C1-C47 alkyl group, a C1-C47 heterocyclic group, or a C6-47 aryl group; and $R^{c2}$ is a hydrogen atom, an optionally substituted C1-C46 alkyl group, an optionally substituted C6-46 aryl group, or an optionally substituted C1-C46 heterocyclic group.

For example, in a second step of the below-described method (i) of producing the PAG compound according to the third aspect of the present invention, specifically in reacting a reaction product obtained in a first step with a hydrophobic group-containing compound containing a glycidyl ether group, a PAG compound in which $R^c$ is a group represented by the formula (3-2) can be produced.

Specific examples of the alkyl, aryl, and heterocyclic groups for $R^{c1}$ are included in those exemplified above.

The number of carbon atoms of the alkyl group for $R^{c1}$ is preferably 4 to 30, more preferably 8 to 20.

The number of carbon atoms of the aryl group for $R^{c1}$ is preferably 6 to 30, more preferably 6 to 25.

The number of carbon atoms of the heterocyclic group for $R^{c1}$ is preferably 1 to 30, more preferably 2 to 20.

When $R^{c2}$ is an optionally substituted C1-C46 alkyl group, an optionally substituted C6-C46 aryl group, or an optionally substituted C1-C46 heterocyclic group, $R^{c2}$ is preferably a group represented by the following formula (3-3) :

$-[CH_2CH(CH_2\text{-}O\text{-}R^{c3})\text{-}O]_j\text{-}H$ (3-3)

wherein $R^{c3}$s are the same as or different from each other and are each C1-C43 alkyl group, a C6-C43 aryl group, or a C1-C43 heterocyclic group; and j is 1 to 10.

For example, in a second step of the below-described method (i) of producing the PAG compound according to the third aspect of the present invention, specifically in reacting a reaction product obtained in a first step with a hydrophobic group-containing compound containing a glycidyl ether group, a PAG compound with j in the formula (3-3) being 1 is produced by reacting the hydroxy group of-$CH_2CH(OH)CH_2\text{-}OR^{c1}$ obtained with one molecule of a hydrophobic group-containing compound containing a glycidyl ether group. Further, a PAG compound with j being 2 is produced by reacting the hydroxy group of the resulting PAG compound with one molecule of a hydrophobic group-containing compound containing a glycidyl ether group.

Specific examples of the alkyl, aryl, and heterocyclic groups for $R^{c3}$ are included in those exemplified above.

The number of carbon atoms of the alkyl group for $R^{c3}$ is preferably 4 to 30, more preferably 8 to 20.

The number of carbon atoms of the aryl group for $R^{c3}$ is preferably 6 to 30, more preferably 6 to 25.

The number of carbon atoms of the heterocyclic group for $R^{c3}$ is preferably 1 to 30, more preferably 2 to 20.

$R^{c3}$ is preferably a C12-C14 alkyl group.

Alternatively, $R^{c3}$ is preferably the same group as $R^{c1}$.

Preferably, j is 1 to 5, more preferably 1 or 2.

In the formula (3-1), $R^c$ is preferably an optionally substituted C1-C50 alkyl group, more preferably a linear or branched unsubstituted aliphatic alkyl group.

In the formula (3-1), $R^2$ is a C21-C100 divalent organic group.

The divalent organic group may be any group and is preferably an optionally substituted divalent hydrocarbon group.

Examples of the divalent hydrocarbon group include a group obtained by abstracting one hydrogen atom from a monovalent hydrocarbon group such as an aliphatic alkyl group, an alicyclic alkyl group, or an aryl group.

Specific examples of the C21-C100 aliphatic alkyl group and the C21-C100 alicyclic alkyl group are included in those exemplified above.

$R^2$ is preferably an optionally substituted aliphatic or alicyclic alkylene group. When $R^2$ contains a substituent, $R^2$ is preferably a group represented by the following formula (3-4):

$-(CH_2)_s\text{-}O\text{-}CH_2CH(OR^{21})CH_2\text{-}$ (3-4)

wherein $R^{21}$ is a C8-C97 organic group; and s is 0 to 10. Preferably, s is 1 to 8, more preferably 2 to 5. $R^2$ is more preferably a group represented by the following formula (3-5):

$-CH_2CH_2\text{-}O\text{-}CH_2CH(OR^{22})CH_2\text{-}$ (3-5)

wherein $R^{22}$ is a C16-C95 organic group.

The C8-C97 organic group for $R^{21}$ in the formula (3-4) and the C16-C95 organic group for $R^{22}$ in the formula (3-5) are each preferably a group represented by the following formula (3-6):

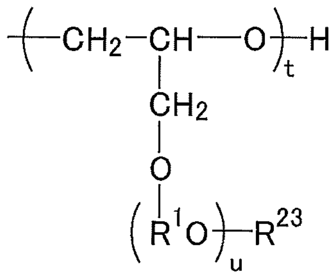

(3-6)

wherein $R^{23}$ is the same as or different from each other and is a C1-C92 alkyl group; t is 1 to 16; $R^1$s are the same as or different from each other and are each a C2-C20 alkylene group; u is the average number of moles of oxyalkylene groups added and is 1 to 46.

$R^1$ is the same as $R^1$ in the formula (1-1).

The number of carbon atoms of the alkyl group for $R^{23}$ is preferably 3 to 18, more preferably 4 to 14, still more preferably 8 to 12.

Alternatively, $R^{23}$ is preferably the same group as $R^c$.

Preferably, t is 1 to 10, more preferably 1 to 5, still more preferably 1 to 3.

Preferably, u is 1 to 30, more preferably 1 to 20, still more preferably 8 to 18.

The PAG compound according to the third aspect of the present invention preferably has a number average molecular weight of 500 to 100000, more preferably 600 to 50000, still more preferably 650 to 10000, particularly preferably 700 to 5000.

The proportion of a group represented by $R^c$ in the PAG compound according to the third aspect of the present invention represented by the formula (3-1) is preferably 2.5 to 60% by mass based on 100% by mass of the PAG compound. This can provide a PAG compound having more suitable hydrophobicity, which has a higher interaction with stains such as sebum. The proportion of a group represented by $R^c$ is more preferably 3 to 50% by mass, still more preferably 4 to 40% by mass.

<Method of producing PAG compound of the present invention>

A PAG compound of the present invention may be produced by any method, and examples of the method include a method (i) including a first step of reacting a compound having a pyrrolidone structure with an alkylene oxide and a second step of reacting the resulting reaction product with a compound containing a hydrophobic group (hydrophobic group-containing compound); and a method (ii) of reacting a compound having a pyrrolidone structure with a compound containing a hydrophobic group at one end of the polyalkylene glycol (hydrophobic group-containing polyalkylene glycol).

The compound having a pyrrolidone structure (hereinafter, also referred to as pyrrolidone structure-containing compound) is preferably a compound represented by the following formula (4):

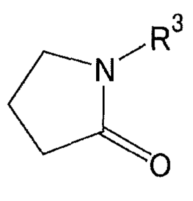

(4)

wherein $R^3$ is a hydrogen atom or a C1-C20 organic group.

In the case of the method (i), the PAG compound according to the first aspect of the present invention is preferably produced by a first step of reacting a pyrrolidone structure-containing compound represented by the formula (4) with an alkylene oxide and a second step of reacting a compound obtained in the first step represented by the following formula (5):

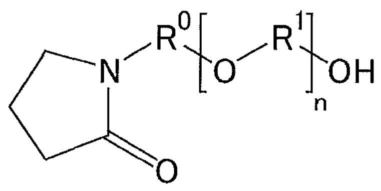

(5)

(wherein $R^0$ is a C1-C20 divalent organic group; $R^1$s are the same as or different from each other and are each a C2-C20 alkylene group; n is the average number of moles of oxyalkylene groups added and is 1 to 300) with a hydrophobic group-containing compound represented by the following formula (6):

$R^4$-$X^1$ (6)

wherein $R^4$ is an optionally substituted C1-C20 alkyl group, an optionally substituted C6-C20 aryl group, or an optionally substituted C1-C20 heterocyclic group; and $X^1$ is a functional group reactive with a hydroxy group.

In the formula (4), $R^3$ is a hydrogen atom or a C1-C20 organic group. In the method (i), the C1-C20 organic group preferably contains a functional group reactive with an alkylene oxide. In the method (ii), the C1-C20 organic group preferably contains a functional group reactive with a compound represented by the below-described formula (7).

Non-limiting examples of these functional groups include polymerizable unsaturated groups such as hydroxy, carboxyl, epoxy, glycidyl, amino, sulfonic acid, phosphoric acid, and vinyl groups. In the method (i), the functional group is preferably a hydroxy, epoxy, or glycidyl group.

The C1-C20 organic group is preferably a group in which any of the above-exemplified functional groups bonds to a hydrocarbon group such as an aliphatic alkyl, alicyclic alkyl, or aryl group. Specific examples of the aliphatic alkyl, alicyclic alkyl, and aryl groups and the ranges of the preferred numbers of carbon atoms thereof are the same as those described for $R^0$ in the formula (1-1).

Specific examples of the pyrrolidone structure-containing compound represented by the formula (4) include N-hydroxyethyl pyrrolidone, N-hydroxypropyl pyrrolidone, N-hydroxybutyl pyrrolidone, N-hydroxypentyl pyrrolidone, and N-hydroxyhexyl pyrrolidone, which are compounds in which a C1-C20 hydroxyalkyl group bonds to the nitrogen atom of 2-pyrrolidone.

The pyrrolidone structure-containing compound represented by the formula (4) is preferably N-hydroxyethyl pyrrolidone, N-hydroxypropyl pyrrolidone, or N-hydroxybutyl pyrrolidone.

In the formula (5), $R^0$ is a C1-C20 divalent organic group, which is produced when $R^3$ in the formula (4) is reacted with an alkylene oxide.

In the formula (6), $R^4$ is an optionally substituted C1-C20 alkyl group, an optionally substituted C6-C20 aryl group, or an optionally substituted C1-C20 heterocyclic group. Specific examples and preferred examples of these groups are the same as those described for $R^a$ in the formula (1-1).

$X^1$ in the formula (6) may be any functional group reactive with the hydroxy group at an end of the polyalkylene glycol of the compound represented by the formula (5), and examples thereof include a halogen atom and epoxy, glycidyl, hydroxy, carboxyl, and amino groups. $X^1$ is preferably a halogen atom or a glycidyl group.

Examples of the halogen atom include fluorine, chlorine, bromine, and iodine atoms. Preferred is a chlorine atom.

Specific examples of the hydrophobic group-containing compound represented by the formula (6) include a hydrophobic group-containing compound containing a halogen atom, such as a C1-C20 halogenated alkyl, and a hydrophobic group-containing compound containing a glycidyl ether group, such as a C1-C20 alkyl glycidyl ether.

Examples of the C1-C20 halogenated alkyl include methyl chloride, ethyl chloride, propyl chloride, butyl chloride, pentyl chloride, hexyl chloride, heptyl chloride, octyl chloride, nonyl chloride, decyl chloride, undecyl chloride, dodecyl chloride (lauryl chloride), tridecyl chloride, tetradecyl chloride, pentadecyl chloride, hexadecyl chloride, heptadecyl chloride, octadecyl chloride, nonadecyl chloride, and eicosanyl chloride.

Preferred among these is a C4-C18 halogenated alkyl. The number of carbon atoms of the halogenated alkyl is more preferably 8 to 16, still more preferably 12 to 14.

Examples of the C1-C20 alkyl glycidyl ether include methyl glycidyl ether, ethyl glycidyl ether, propyl glycidyl ether, butyl glycidyl ether, pentyl glycidyl ether, hexyl glycidyl ether, heptyl glycidyl ether, octyl glycidyl ether, nonyl glycidyl ether, decyl glycidyl ether, undecyl glycidyl ether, dodecyl glycidyl ether, tridecyl glycidyl ether, tetradecyl glycidyl ether, pentadecyl glycidyl ether, hexadecyl glycidyl ether, and heptadecyl glycidyl ether.

Preferred among these is a C7-C19 alkyl glycidyl ether. The number of carbon atoms of the alkyl glycidyl ether is more preferably 11 to 18, still more preferably 15 to 17.

Reaction of the compound represented by the formula (5) with a hydrophobic group-containing compound containing a glycidyl ether group gives first a compound represented by the formula (1-1) in which $R^a$ is $-CH_2CH(OH)CH_2-OR^{a1}$. This compound represented by the formula (1-1) contains a hydroxy group. Reaction of this

hydroxy group with a hydrophobic group-containing compound containing a glycidyl ether group provides a structure in which $R^a$ is a group represented by the formula (1-2) with $R^{a2}$ being a group represented by the formula (1-3).

In the case of the method (ii), the PAG compound according to the first aspect of the present invention is preferably produced by reacting a pyrrolidone structure-containing compound represented by the formula (4) with a compound represented by the following formula (7):

$$X^2\text{-}(OR^1)_n\text{-}O\text{-}R^a \qquad (7)$$

wherein $R^a$ is an optionally substituted C1-C20 alkyl group, an optionally substituted C6-C20 aryl group, or an optionally substituted C1-C20 heterocyclic group; $R^1$s are the same as or different from each other and are each a C2-C20 alkylene group; n is the average number of moles of oxyalkylene groups added and is 1 to 300; and $X^2$ is a hydrogen atom or a reactive functional group.

$R^1$, $R^a$, and n in the formula (7) are the same as $R^1$, $R^a$, and n in the formula (1-1).

In the formula (7), $X^2$ is a hydrogen atom or a reactive functional group and may be any one reactive with the organic group for $R^3$ in the pyrrolidone structure-containing compound represented by the formula (4). Examples of the reactive functional group include a halogen atom, a glycidyl group, a hydroxy group, a carboxyl group, an amino group, and a thiol group-terminated C1-C10 organic group. $X^2$ is preferably a hydrogen atom, a halogen atom, a glycidyl group, or a thiol group-terminated C1-C10 organic group, more preferably a hydrogen atom, a glycidyl group, or a thiol group-terminated C1-C10 organic group.

Specific examples of the compound represented by the formula (7) containing a glycidyl group include an alkoxy polyalkylene glycol glycidyl ether containing a C1-C20 alkoxy group.

Examples of the alkoxy group of the alkoxy polyalkylene glycol glycidyl ether include methoxy, ethoxy, propoxy, butoxy, pentyloxy, hexyloxy, heptyloxy, octyloxy, nonyloxy, decyloxy, undecyloxy, dodecyloxy (lauryloxy), tridecyloxy, tetradecyloxy, pentadecyloxy, hexadecyloxy, heptadecyloxy, octadecyloxy, nonadecyloxy, and eicosanyloxy groups.

In the method (ii), reaction of the pyrrolidone structure-containing compound represented by the formula (4) with an alkoxy polyalkylene glycol glycidyl ether gives first a compound represented by the formula (1-1) in which $R^0$ is a group represented by the formula (1-6). This compound represented by the formula (1-1) contains a hydroxy group. Reaction of this hydroxy group with an alkoxy polyalkylene glycol glycidyl ether provides a structure in which $R^0$ is a group represented by the formula (1-4) with $R^{01}$ being a group represented by the formula (1-5) .

The thiol group-terminated C1-C10 organic group for $X^2$ is preferably a group represented by the following formula (7-1):

$$-R^{04}\text{-}SH \qquad (7\text{-}1)$$

wherein $R^{04}$ is an optionally substituted C1-C10 alkylene group. The substituent is preferably, for example, a carbonyl, hydroxy, carboxyl, or sulfonic acid group, with a carbonyl group being more preferred.

The group represented by the formula (7-1) is more preferably a group represented by the following formula (7-2) :

$$-CO\text{-}R^{06}\text{-}SH \qquad (7\text{-}2)$$

(wherein $R^{06}$ is a C1-C9 alkylene group), still more preferably a group represented by the following formula (7-3) :

$$-CO\text{-}CH_2CH_2\text{-}SH \qquad (7\text{-}3).$$

$R^{04}$ and $R^{06}$ are the same as $R^{04}$ in the formula (1-8) and $R^{06}$ in the formula (1-9), respectively.

A compound represented by the formula (7) in which $X^2$ is a group represented by the formula (7-2) can be obtained by reacting a mercaptocarboxylic acid with a compound represented by the formula (7) in which $X^2$ is a hydrogen atom. Specific examples of the mercaptocarboxylic acid include thioglycolic acid (mercaptoacetic acid), 3-mercaptopropionic acid, 2-mercaptopropionic acid (thiolactic acid), 4-mercaptobutanoic acid, and thiomalic acid. Preferred is 3-mercaptopropionic acid.

In the method (ii), when the compound represented by the formula (7) in which $X^2$ is a hydrogen atom is used, the pyrrolidone structure-containing compound represented by the formula (4) has only to have a functional group reactive with the hydroxy group at an end of the polyalkylene glycol of the compound represented by the formula (7). The pyrrolidone structure-containing compound preferably contains an epoxy group.

The pyrrolidone structure-containing compound containing an epoxy group is preferably a compound represented by the following formula (4-1):

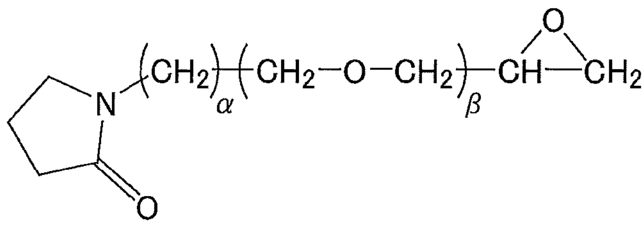

(4-1)

wherein $\alpha$ is 0 to 5; and $\beta$ is 0 or 1.

In the formula (4-1), $\alpha$ is preferably 0 to 3, more preferably 0 to 2, still more preferably 0 or 1.

In the formula (4-1), a combination of $\alpha$ and $\beta$ ($\alpha$, $\beta$) is preferably (1, 1) or (1, 0).

The compound represented by the formula (4-1) can be produced, for example, by reacting a pyrrolidone structure-containing compound such as N-hydroxyethyl pyrrolidone, N-hydroxypropyl pyrrolidone, N-hydroxybutyl pyrrolidone, N-hydroxypentyl pyrrolidone, N-hydroxyhexyl pyrrolidone, or 2-pyrrolidone with a compound containing an epoxy group and a functional group reactive with a nitrogen atom or a hydrogen atom of the pyrrolidone structure-containing compound. Examples of the functional group reactive with a nitrogen atom or a hydrogen atom include an epoxy group, a halogen atom, a hydroxy group, a carboxyl group, and an amino group. Preferred is a halogen atom. The compound containing an epoxy group and a halogen atom is preferably an epihalohydrin, more preferably epichlorohydrin.

In the method (ii), when the compound represented by the formula (7) in which $X^2$ is a thiol group-terminated C1-C10 organic group is used, the pyrrolidone structure-containing compound represented by the formula (4) preferably contains a polymerizable unsaturated group such as a vinyl group, more preferably contains a vinyl group. Specific examples of the pyrrolidone structure-containing compound represented by the formula (4) include N-vinylpyrrolidone and 1-(2-propenyl)-2-pyrrolidone. Preferred is N-vinylpyrrolidone.

The PAG compound according to the second aspect of the present invention can also be produced by the method (i) or (ii), and is preferably produced by the method (i).

In the case of the method (i), the PAG compound according to the second aspect of the present invention is preferably produced by a first step similar to that in the production of the PAG compound according to the first aspect of the present invention and a second step of reacting a compound represented by the formula (5) with a compound represented by the following (8):

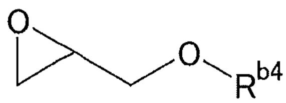

(8)

wherein $R^{b4}$ is a C18-C47 organic group.

$R^{b4}$ in the formula (8) is the same as $R^{b1}$ in the case where $R^{b2}$ in the formula (2-2) is a hydrogen atom.

Reaction of the compound represented by the formula (5) with a compound represented by the formula (8) gives first a compound represented by the formula (2-1) in which $R^b$ is a group represented by the formula (2-2) with $R^{b2}$ being a hydrogen atom. This compound represented by the formula (2-1) contains a hydroxy group. Reaction of this hydroxy group with a compound represented by the formula (8) provides a structure in which $R^{b2}$ in the formula (2-2) is a group represented by the formula (2-3).

The PAG compound according to the third aspect of the present invention can also be produced by the method (i) or (ii). In the case of the method (i), the PAG compound according to the third aspect of the present invention is preferably produced by reacting a compound represented by the following formula (9):

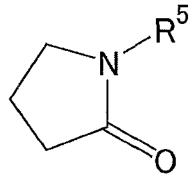

(9)

(wherein $R^5$ is a C21-C100 organic group) with an alkylene oxide.

In the formula (9), $R^5$ is a C21-C100 organic group and preferably contains a functional group reactive with an alkylene oxide. Examples of the functional group include the functional groups exemplified for $R^3$ in the formula (4). The

functional group is preferably a hydroxy group.

The C21-C100 organic group is preferably a group in which any of the above-exemplified functional groups bonds to a hydrocarbon group such as an aliphatic alkyl, alicyclic alkyl, or aryl group. Specific examples and preferred examples of the aliphatic alkyl, alicyclic alkyl, and aryl groups are the same as the aliphatic alkyl, alicyclic alkyl, and aryl groups described for $R^2$ in the formula (3-1).

Specific examples of the compound represented by the formula (9) include N-hydroxyhenicosyl pyrrolidone, N-hydroxydocosyl pyrrolidone, N-hydroxypentacosyl pyrrolidone, and N-hydroxytriacontyl pyrrolidone, which are compounds in which a C21-C100 hydroxyalkyl group bonds to the nitrogen atom of 2-pyrrolidone.

In the second step in the method (i), the reaction product obtained in the first step is preferably reacted with a compound represented by the formula (6).

The PAG compound according to the third aspect of the present invention is preferably produced by the method (ii). In the method (ii), a compound represented by the formula (10) :

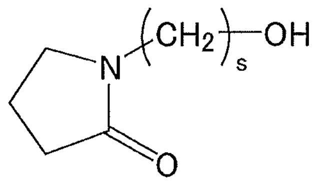

(10)

(wherein s is 0 to 10) is preferably reacted with a compound represented by the following formula (11):

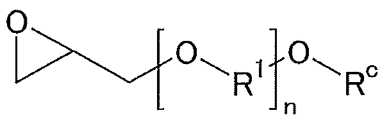

(11)

wherein $R^1$s are the same as or different from each other and are each a C2-C20 alkylene group; $R^c$ is an optionally substituted C1-C50 alkyl group, an optionally substituted C6-C50 aryl group, or an optionally substituted C1-C50 heterocyclic group; and n is the average number of moles of oxyalkylene groups added and is 1 to 300.

s in the formula (10) is the same as s in the formula (3-4).

$R^1$, $R^c$, and n in the formula (11) are the same as $R^1$, $R^c$, and n in the formula (3-1).

In the reaction step in the method (ii), reaction of the compound represented by the formula (10) with a compound represented by the formula (11) gives first a compound represented by the formula (3-1) in which $R^2$ is-$(CH_2)_s$-O-$CH_2CH(OH)CH_2$-. This compound represented by the formula (3-1) contains a hydroxy group. Reaction of this hydroxy group with a compound represented by the formula (11) provides a structure in which $R^2$ in the formula (3-1) is a group represented by the formula (3-5) with $R^{22}$ being a group represented by the formula (3-6).

When a PAG compound of the present invention is produced by the method (i), any amount of the hydrophobic group-containing compound may be used in the second step. The amount thereof is preferably 90 to 500 mol%, more preferably 100 to 300 mol% relative to 100 mol% of the reaction product obtained in the first step.

When the PAG compounds according to the first and second aspects of the present invention are produced by the method (ii), the amount of the hydrophobic group-containing polyalkylene glycol used is preferably 90 to 500 mol%, more preferably 100 to 300 mol% relative to 100 mol% of the pyrrolidone structure-containing compound.

When the PAG compound according to the third aspect of the present invention is produced by the method (ii), the amount of the hydrophobic group-containing polyalkylene glycol used is preferably 90 to 1000 mol%, more preferably 100 to 500 mol% relative to 100 mol% of the pyrrolidone structure-containing compound.

In the first and second steps in the method (i) and the reaction step in the method (ii) for producing a PAG compound of the present invention, a reaction catalyst is preferably used in terms of reaction rate. Examples of the reaction catalyst include acid catalysts such as mineral acids (e.g., sulfuric acid, phosphoric acid) and Lewis acids (e.g., tin tetrachloride, boron trifluoride); basic catalysts such as alkali metal hydroxides (e.g., sodium hydroxide, potassium hydroxide); and polymerization catalysts. Preferred are basic catalysts and polymerization catalysts.

Examples the polymerization catalysts include persulfates such as ammonium persulfate, sodium persulfate, and potassium persulfate; hydrogen peroxide; and watersoluble azo initiators such as azoamidine compounds (e.g., 2,2'-azobis-2-methylpropionamidine hydrochloride), cyclic azoamidine compounds (e.g., 2,2'-azobis-2-(2-imidazolin-2-yl)propane hydrochloride), and azonitrile compounds (e.g., 2-carbamoyl azoisobutyronitrile).

A basic catalyst is preferably used in the first and second steps in the method (i) or in the case of using a hydroxy group-terminated polyalkylene glycol compound or a glycidyl group-containing compound as a (poly)alkylene glycol compound in the method (ii). The basic catalyst is more preferably sodium hydroxide.

In the case of using a thiol group-terminated (poly)alkylene glycol compound in the method (ii), a watersoluble azo initiator is preferably used. The watersoluble azo initiator is more preferably an azoamidine compound.

The reaction temperature in each of the first and second steps in the method (i) and the reaction step in the method (ii) may be any temperature and is preferably 20°C to 180°C, more preferably 30°C to 160°C. The reaction pressure is preferably from an atmospheric pressure to 20 $kg/cm^2G$, more preferably 1 to 10 $kg/cm^2G$.

The first and second steps in the method (i) and the reaction step in the method (ii) are preferably performed under an inert gas atmosphere such as a nitrogen atmosphere. Further, the resulting reaction product may be stored under an inert gas atmosphere such as a nitrogen atmosphere.

A solvent may be used in these steps. Preferably, no solvent is used for the reactions.

The methods of producing a PAG compound of the present invention may include purification during or after the first step and/or the second step for the method (i) and the reaction step for the method (ii), if necessary.

Examples of the purification include removal of components such as residual materials, by-products, moisture, and residual catalysts and neutralization of catalysts.

In the neutralization of catalysts, for example, when sodium hydroxide and/or potassium hydroxide are/is used for reaction, a neutralizing agent used may be sulfuric acid, acetic acid, or a solid acid such as activated white earth.

<Composition containing PAG compound of the present invention>

A composition containing a PAG compound of the present invention essentially contains a PAG compound of the present invention. The composition may contain in addition to a PAG compound of the present invention different components such as unreacted materials, by-products, residual catalysts, and solvents.

A PAG compound of the present invention is preferably present in the composition in an amount of 1 to 99.9% by mass, more preferably 1 to 99% by mass based on 100% by mass of the composition.

Specifically, the composition containing any of the polyalkylene glycol-containing compounds of the present invention contains a polyalkylene glycol-containing compound and different components other than the compounds. The amount of the polyalkylene glycol-containing compound is 1 to 99.9% by mass, preferably 30 to 99.9% by mass, still more preferably 50 to 99.9% by mass based on 100% by mass of the composition.

<Uses of PAG compound of the present invention>

The polyalkylene glycol-containing compounds of the present invention are used in various applications such as detergent builders, detergents, chemicals for water treatment, scale inhibitors, and dispersants, and preferably used for detergent builders and detergents, for example. The present invention also encompasses a method of using the polyalkylene glycol-containing compounds as detergent builders, detergent additives, chemicals for water treatment, scale inhibitors, or dispersants.

The present invention also relates to a detergent composition containing any of the polyalkylene glycol-containing compounds of the present invention and a detergent additive other than the compounds.

The detergent additive other than the polyalkylene glycol-containing compounds of the present invention may be any additive that is used for surfactants and common detergents. The detergent additive may be selected by appropriately referring to common knowledge in the detergent field.

The detergent composition may also be a powder detergent composition or a liquid detergent composition.

The surfactant is preferably one or more surfactants selected from the group consisting of anionic surfactants, nonionic surfactants, cationic surfactants, and amphoteric surfactants.

Suitable examples of the anionic surfactants include alkylbenzene sulfonates, alkyl ether sulfates, alkenyl ether sulfates, alkyl sulfates, alkenyl sulfates, α-olefin sulfonates, α-sulfonated fatty acids and esters thereof, alkane sulfonates, saturated fatty acid salts, unsaturated fatty acid salts, alkyl ether carboxylates, alkenyl ether carboxylates, amino acid-based surfactants, N-acylamino acid-based surfactants, alkyl phosphoric acid esters and salts thereof, and alkenyl phosphoric acid esters and salts thereof. The alkyl group or alkenyl group of any of these anionic surfactants may contain an alkyl group such as a methyl group as a branch.

Suitable examples of the nonionic surfactants include polyoxyalkylene alkyl ethers, polyoxyalkylene alkenyl ethers, polyoxyethylene alkylphenyl ethers, higher fatty acid alkanolamides and adducts thereof with an alkylene oxide, sucrose fatty acid esters, alkyl glycoxides, fatty acid glycerol monoesters, and alkylamine oxides. The alkyl group or alkenyl group of any of these nonionic surfactants may contain an alkyl group such as a methyl group as a branch.

Suitable examples of the cationic surfactants include quaternary ammonium salts. Suitable examples of the amphoteric surfactants include carboxyl-type amphoteric surfactants and sulfobetaine-type amphoteric surfactants. The alkyl group or alkenyl group of any of these cationic surfactants and amphoteric surfactants may contain an alkyl group such as a methyl group as a branch.

The surfactant is typically present in the detergent composition in a proportion of 10 to 60% by mass, preferably

15 to 50% by mass, more preferably 20 to 45% by mass, particularly preferably 25 to 40% by mass based on the total amount of the detergent composition. A detergent composition containing too small an amount of the surfactant may fail to exhibit sufficient detergency, whereas a detergent composition containing too large an amount of the surfactant may be disadvantageous in terms of cost.

EXAMPLES

The present invention is described in further detail below with reference to examples, but the present invention is not limited to these examples. It should be noted that the terms "part(s)" and "%" refer to "part(s) by mass" and "% by mass", respectively, unless otherwise stated.

<Evaluation of composite stain (oil/protein/mud/carbon black) detergency>

(1) The whiteness of 5 cm × 5 cm artificially stained wet fabrics (available from Sentaku Kagaku Kyokai) was measured in advance using a color difference meter SE 6000 (Nippon Denshoku Industries Co., Ltd.) based on the reflectance.
(2) Pure water was added to calcium chloride dihydrate (7.34 g) to prepare 20 kg of hard water.
(3) In a 20-mL vial, 10 g of an aqueous solution of each sample to be evaluated (solid content: 2%) was prepared.
(4) Sodium laurylbenzenesulfonate (0.5 g), sodium sulfate pure water (2 g), and sodium carbonate (0.5 g) as detergent components were weighed and put into a beaker.
(5) To the beaker in (4) was added the hard water prepared in (2) to prepare 2 kg of a wash solution.
(6) A Terg-o-Tometer was set at 25°C, and each pot was charged with the wash solution (500 mL) prepared in (5) and the aqueous solution (1.0 g) of each sample prepared in (3). The contents were stirred at 100 rpm for two minutes. Thereafter, five stained fabrics and 14.6 g of a cotton fabric (5 cm × 5 cm CW98 available from Testfabrics, Inc.) for liquor ratio control were put into each pot, and they were stirred at 100 rpm for 10 minutes (a reference was also prepared in which pure water was added instead of the aqueous solution of each sample prepared in (3) and was evaluated).
(7) The stained fabrics taken out from the pots were wrung out by hand to remove water. The hard water (500 mL) prepared in (2) was put into each pot, controlled to 25°C, and stirred at 100 rpm for four minutes.
(8) The stained fabrics taken out from the pots were wrung out by hand to remove water and dried at room temperature overnight. Thereafter, the whiteness of the stained fabrics was measured again using the color difference meter based on the reflectance.
(9) The whiteness of the CW98 as an unstained fabric (hereinafter, also referred to as white fabric) was measured based on the reflectance as in (8).
(10) A washing percentage (detergency) was determined from the resulting measurement values using the following equation:

Washing percentage (%) = ((whiteness of stained fabric before washing) - (whiteness of stained fabric after washing))/((whiteness of stained fabric before washing) - (whiteness of white fabric)) × 100.

A higher washing percentage indicates better detergency.

<Evaluation of composite stain (clay-carbon black) anti-redeposition ability>

(1) A cotton fabric (CW98 available from Testfabrics, Inc.) was cut to prepare 5 cm × 5 cm white fabrics. The whiteness of the white fabrics was measured in advance using a color difference meter SE 6000 (Nippon Denshoku Industries Co., Ltd.) based on the reflectance.
(2) Pure water was added to calcium chloride dihydrate (1.47 g) to prepare 20 kg of hard water.
(3) In a 20-mL vial, 10 g of an aqueous solution of each sample to be evaluated (solid content: 1%) was prepared.
(4) 100 g of 4% sodium laurylbenzenesulfonate was prepared as an aqueous surfactant solution.

(5) A Terg-o-Tometer was set at 25°C, and each pot was charged with the hard water (1 L) prepared in (2), the aqueous solution (5.0 g) of each sample prepared in (3), the aqueous surfactant solution (5.0 g) prepared in (4), carbon black (0.25 g) (available from Sentaku Kagaku Kyokai), and clay (0.50 g) (JIS test powder 1, class 11 (calcined KANTO loam)). The contents were stirred at 100 rpm for one minute. Thereafter, five cotton fabrics were put into each pot, and they were stirred at 100 rpm for 10 minutes (a reference was also prepared in which pure water was added instead of the aqueous solution of each sample prepared in (3) and was evaluated).

(6) The white fabrics were wrung out by hand to remove water. The hard water (1 L) prepared in (2) was put into each pot, controlled to 25°C, and stirred at 100 rpm for two minutes.

(7) The white fabrics were covered with an ironing fabric and smoothed with an iron to dry. Thereafter, the whiteness of the cotton fabrics was again measured using the color difference meter based on the reflectance.

(8) An anti-redeposition percentage (anti-redeposition ability) was determined from the resulting measurement values using the following equation:

$$\text{Anti-redeposition percentage (\%)} = \text{(whiteness after washing)}/\text{(whiteness before washing)} \times 100$$

A higher anti-redeposition percentage indicates better anti-redeposition ability.

<Evaluation of oil-water interfacial tension lowering properties>

With a lower oil-water interfacial tension, oil has a higher affinity for water and is more easily dispersed in washing water. This leads to improvement of detergency. The oil-water interfacial tension was measured using a DY-500 system (hereinafter, also referred to as DY-500, available from Kyowa Interface Science Co., Ltd.) in accordance with the following procedure.

(1) Pure water was added to sodium laurylbenzenesulfonate (0.4 g) to prepare 1000 g of an aqueous sodium laurylbenzenesulfonate solution (400 ppm).

(2) In a 20-mL vial, 10 g of an aqueous solution of each sample to be evaluated (solid content: 2%) was prepared.

(3) To the aqueous solution (0.16 g) of each sample prepared in (2) was added the aqueous sodium laurylbenzenesulfonate solution prepared in (1) to prepare 80.0 g of an aqueous phase solution.

(4) A glass vessel was charged with the solution (20 g) prepared in (3). To the vessel was added paraffin oil (30.0 g) gently using a dropper as an oil phase solution. Thus, an oil-water two-phase solution was prepared.

(5) The two-phase solution prepared in (4) was controlled to 25°C and allowed to stand for 10 minutes while the temperature was maintained at 25°C.

(6) After the solution was allowed to stand, it was sampled and subjected to measurement of oil-water interfacial tension using a platinum plate.

<Evaluation of strength of interaction with stains>

The strength of interaction between each sample and each stain component was evaluated by measuring the absolute values of heat changes that occurred when an aqueous solution of each sample was added dropwise to an aqueous solution of a stain component using a MicroCal ITC 200 system (Malvern Panalytical Ltd., hereinafter, also referred to as ITC).

(1) In a 20-mL vial, 10 g of an aqueous solution of each sample to be evaluated (solid content: 800 ppm) was prepared.

(2) Separately, in a 10-mL vial, 5 g of an aqueous solution of each stain component (solid content: 1%) was prepared. Each stain component as a model stain component was selected from methyl acetate, acetic acid, and alkyl fatty acids.

(3) The aqueous solution of each sample to be evaluated prepared in (1) was put into a sample cell of the ITC, the aqueous solution of a stain component prepared in (2) was put into a syringe, and the titration was started. The measurement conditions were as follows: the total number of injections was 19, the cell temperature was 25°C, the reference power during measurement was 5 μcal, the duration from the start of data capturing to the 1st injection (initial delay) was 60 seconds, the stirring speed was 750 rpm, each injection volume was 0.4 μL for the 1st injection and 2 μL for the 2nd to 19th injections, the duration of each injection was four seconds, the spacing between injections was 150 seconds, and the data averaging time (filter period) was five seconds. A reference was also prepared in which pure water was added instead of the solution of each sample prepared in (1) and was subjected to measurement.

(4) The sum of the maximum values of the absolute values of heat changes occurred at the 2nd to 19th injections

was determined as the strength of interaction. This means that a larger sum of the maximum values indicates stronger interaction.

<Example 1>

A stainless steel autoclave reactor equipped with a thermometer, a stirrer, a material inlet tube, and a nitrogen inlet tube was charged with 374.6 g (2.9 mol) of N-hydroxyethyl pyrrolidone (hereinafter, also referred to as HEP) and 0.33 g of sodium hydroxide as a reaction catalyst. The reactor was purged with nitrogen under stirring, and the contents were heated to 120°C in a nitrogen atmosphere. Then, 1281.8 g (29.0 mol) of ethylene oxide was introduced into the reactor over 3.5 hours at a safe pressure while the temperature was maintained at 120°C ± 5°C. Thereafter, the temperature was maintained for two hours. Finally, the reactor was purged with nitrogen under stirring, and the contents were cooled to room temperature. Thus, a polyalkylene glycol-containing compound (1) in which an average of 10 mol of ethylene oxide was added to HEP was obtained. Next, a four-necked glass recovery flask reaction vessel equipped with a thermometer, a reflux tube, and a nitrogen inlet tube was charged with 2.91 g (5.1 mmol) of the polyalkylene glycol-containing compound (1), 0.30 g (7.5 mmol) of sodium hydroxide as a reaction catalyst, and a stir bar. The reaction vessel was purged with nitrogen under stirring using a magnetic stirrer, and the contents were heated to 80°C in a nitrogen atmosphere. While the temperature was maintained at 80°C under atmospheric pressure, 1.36 g (6.6 mmol) of 1-lauryl chloride was added to the reaction vessel in one portion, and then the temperature was maintained for 7.5 hours in a nitrogen atmosphere. Thereafter, the reaction solution was cooled to room temperature, and ion exchange water was added thereto, followed by sufficient stirring. The resulting dispersion solution was put into a separatory funnel and washed with n-hexane, and then the lower phase was drained to collect the aqueous phase. This washing operation was repeated three times. The collected aqueous phase was subjected to evaporation to remove the solvent. To the residue was added acetonitrile, and insoluble matter was separated by centrifugation. After the centrifugation, the supernatant was collected and subjected to evaporation to remove acetonitrile. Thus, an organic group-terminated polyalkylene glycol-containing compound (4) was obtained.

<Example 2>

A stainless steel autoclave reactor equipped with a thermometer, a stirrer, a material inlet tube, and a nitrogen inlet tube was charged with 600 g (1.1 mol) of the polyalkylene glycol-containing compound (1). The reactor was purged with nitrogen under stirring, and the contents were heated to 120°C in a nitrogen atmosphere. Then, 195.1 g (4.4 mol) of ethylene oxide was introduced into the reactor over 2.5 hours at a safe pressure while the temperature was maintained at 120°C ± 5°C. Thereafter, the temperature was maintained for 1.5 hours. Finally, the reactor was purged with nitrogen under stirring, and the contents were cooled to room temperature. Thus, a polyalkylene glycol-containing compound (2) in which an average of 14.2 mol of ethylene oxide was added to HEP was obtained. Separately, a stainless steel autoclave reactor equipped with a thermometer, a stirrer, a material inlet tube, and a nitrogen inlet tube was charged with 780.7 g (1.1 mol) of the resulting polyalkylene glycol-containing compound (2). The reactor was purged with nitrogen under stirring, and the contents were heated to 120°C in a nitrogen atmosphere. Then, 284.2 g (6.5 mol) of ethylene oxide was introduced into the reactor over 5.3 hours at a safe pressure while the temperature was maintained at 120°C ± 5°C. Thereafter, the temperature was maintained for 4.2 hours. Finally, the reactor was purged with nitrogen under stirring, and the contents were cooled to room temperature. Thus, a polyalkylene glycol-containing compound (3) in which an average of 20.4 mol of ethylene oxide was added to HEP was obtained. Next, a four-necked glass recovery flask reaction vessel equipped with a thermometer, a reflux tube, and a nitrogen inlet tube was charged with 2.25 g (2.2 mmol) of the polyalkylene glycol-containing compound (3), 0.13 g (3.2 mmol) of sodium hydroxide as a reaction catalyst, and a stirring bar. The reaction vessel was purged with nitrogen under stirring using a magnetic stirrer, and the contents were heated to 80°C in a nitrogen atmosphere. While the temperature was maintained at 80°C under atmospheric pressure, 0.60 g (2.9 mmol) of 1-lauryl chloride was added to the reaction vessel in one portion, and then the temperature was maintained for 7.5 hours in a nitrogen atmosphere. Thereafter, the reaction solution was cooled to room temperature, and ion exchange water was added thereto, followed by sufficient stirring. The resulting dispersion solution was put into a separatory funnel and washed with n-hexane, and then the lower phase was drained to collect the aqueous phase. This washing operation was repeated three times. The collected aqueous phase was subjected to evaporation to remove the solvent. To the residue was added acetonitrile, and insoluble matter was separated by centrifugation. After the centrifugation, the supernatant was collected and subjected to evaporation to remove acetonitrile. Thus, an organic group-terminated polyalkylene glycol-containing compound (5) was obtained.

<Example 3-1>

A four-necked glass recovery flask reaction vessel equipped with a thermometer, a stirrer, a reflux tube, and

a nitrogen inlet tube was charged with 2.91 g (5.1 mmol) of the polyalkylene glycol-containing compound (1) and 0.30 g (7.5 mmol) of sodium hydroxide as a reaction catalyst. The reaction vessel was purged with nitrogen under stirring, and the contents were heated to 60°C in a nitrogen atmosphere. While the temperature was maintained at 60°C under atmospheric pressure, 1.23 g (5.1 mmol) of lauryl glycidyl ether was added to the reaction vessel in one portion, and then the temperature was maintained for 10 hours in a nitrogen atmosphere. Thereafter, the reaction solution was cooled to room temperature. Thus, an organic group-terminated polyalkylene glycol-containing compound (6-1) was obtained.

<Example 4-1>

A four-necked glass recovery flask reaction vessel equipped with a thermometer, a stirrer, a reflux tube, and a nitrogen inlet tube was charged with 2.91 g (5.1 mmol) of the polyalkylene glycol-containing compound (1) and 0.30 g (7.5 mmol) of sodium hydroxide as a reaction catalyst. The reaction vessel was purged with nitrogen under stirring, and the contents were heated to 60°C in a nitrogen atmosphere. While the temperature was maintained at 60°C under atmospheric pressure, 0.95 g (5.1 mmol) of 2-ethylhexyl glycidyl ether was added to the reaction vessel in one portion, and then the temperature was maintained for 10 hours in a nitrogen atmosphere. Thereafter, the reaction solution was cooled to room temperature. Thus, an organic group-terminated polyalkylene glycol-containing compound (7-1) was obtained.

<Example 5-1>

A four-necked glass recovery flask reaction vessel equipped with a thermometer, a stirrer, a reflux tube, and a nitrogen inlet tube was charged with 2.91 g (5.1 mmol) of the polyalkylene glycol-containing compound (1) and 0.30 g (7.5 mmol) of sodium hydroxide as a reaction catalyst. The reaction vessel was purged with nitrogen under stirring, and the contents were heated to 60°C in a nitrogen atmosphere. While the temperature was maintained at 60°C under atmospheric pressure, 1.67 g (5.1 mmol) of stearyl glycidyl ether was added to the reaction vessel in one portion, and then the temperature was maintained for 12 hours in a nitrogen atmosphere. Thereafter, the reaction solution was cooled to room temperature. Thus, an organic group-terminated polyalkylene glycol-containing compound (8-1) was obtained.

<Example 6-1>

A four-necked glass recovery flask reaction vessel equipped with a thermometer, a stirrer, a reflux tube, and a nitrogen inlet tube was charged with 5.15 g (5.1 mmol) of the polyalkylene glycol-containing compound (3) and 0.30 g (7.5 mmol) of sodium hydroxide as a reaction catalyst. The reaction vessel was purged with nitrogen under stirring, and the contents were heated to 60°C in a nitrogen atmosphere. While the temperature was maintained at 60°C under atmospheric pressure, 1.23 g (5.1 mmol) of lauryl glycidyl ether was added to the reaction vessel in one portion, and then the temperature was maintained for 12 hours in a nitrogen atmosphere. Thereafter, the reaction solution was cooled to room temperature. Thus, an organic group-terminated polyalkylene glycol-containing compound (9-1) was obtained.

<Example 7-1>

A four-necked glass recovery flask reaction vessel equipped with a thermometer, a stirrer, a reflux tube, and a nitrogen inlet tube was charged with 0.66 g (5.1 mmol) of N-hydroxyethyl pyrrolidone (hereinafter, also referred to as HEP) and 0.30 g (7.5 mmol) of sodium hydroxide as a reaction catalyst. The reaction vessel was purged with nitrogen under stirring, and the contents were heated to 60°C in a nitrogen atmosphere. While the temperature was maintained at 60°C under atmospheric pressure, 4.60 g (5.1 mmol) of Denacol EX-171 (available from NCX, lauryl alcohol-EO(15)-glycidyl ether) was added to the reaction vessel in one portion, and then the temperature was maintained for 12 hours in a nitrogen atmosphere. Thereafter, the reaction solution was cooled to room temperature. Thus, an organic group-terminated polyalkylene glycol-containing compound (10-1) was obtained.

<Example 8-1>

A four-necked glass recovery flask reaction vessel equipped with a thermometer, a stirrer, a reflux tube, and a nitrogen inlet tube was charged with 0.66 g (5.1 mmol) of N-hydroxyethyl pyrrolidone (hereinafter, also referred to as HEP) and 0.30 g (7.5 mmol) of sodium hydroxide as a reaction catalyst. The reaction vessel was purged with nitrogen under stirring, and the contents were heated to 60°C in a nitrogen atmosphere. While the temperature was maintained at 60°C under atmospheric pressure, 13.8 g (15.3 mmol) of Denacol EX-171 (available from NCX, lauryl alcohol-EO(15)-glycidyl ether) was added to the reaction vessel over five minutes, and then the temperature was maintained for 24 hours in a nitrogen atmosphere. Thereafter, the reaction solution was cooled to room temperature. Thus, an

organic group-terminated polyalkylene glycol-containing compound (11-1) was obtained.

<Example 3-2>

A four-necked glass recovery flask reaction vessel equipped with a thermometer, a stirrer, and a nitrogen inlet tube was charged with 11.38 g (20 mmol) of the polyalkylene glycol-containing compound (1) and 0.52 g of sodium hydroxide as a catalyst. The reaction vessel was purged with nitrogen under stirring, and the contents were heated to 120°C in a nitrogen atmosphere. The temperature was maintained for one hour under atmospheric pressure. Thereafter, the temperature was lowered to 80°C, 5.12 g (20 mmol) of lauryl glycidyl ether was added to the reaction vessel in one portion, and then the temperature was maintained for seven hours in a nitrogen atmosphere. Thereafter, the reaction solution was cooled to room temperature. Thus, an organic group-terminated polyalkylene glycol-containing compound (6-2) was obtained.

<Example 4-2>

A four-necked glass recovery flask reaction vessel equipped with a thermometer, a stirrer, and a nitrogen inlet tube was charged with 11.38 g (20 mmol) of the polyalkylene glycol-containing compound (1) and 0.52 g of sodium hydroxide as a catalyst. The reaction vessel was purged with nitrogen under stirring, and the contents were heated to 120°C in a nitrogen atmosphere. The temperature was maintained for one hour under atmospheric pressure. Thereafter, the temperature was lowered to 80°C, 3.73 g (20 mmol) of 2-ethylhexyl glycidyl ether was added to the reaction vessel in one portion, and then the temperature was maintained for seven hours in a nitrogen atmosphere. Thereafter, the reaction solution was cooled to room temperature. Thus, an organic group-terminated polyalkylene glycol-containing compound (7-2) was obtained.

<Example 5-2>

A four-necked glass recovery flask reaction vessel equipped with a thermometer, a stirrer, and a nitrogen inlet tube was charged with 11.38 g (20 mmol) of the polyalkylene glycol-containing compound (1) and 0.52 g of sodium hydroxide as a catalyst. The reaction vessel was purged with nitrogen under stirring, and the contents were heated to 120°C in a nitrogen atmosphere. The temperature was maintained for one hour under atmospheric pressure. Thereafter, the temperature was lowered to 80°C, 6.53 g (20 mmol) of stearyl glycidyl ether was added to the reaction vessel in one portion, and then the temperature was maintained for seven hours in a nitrogen atmosphere. Thereafter, the reaction solution was cooled to room temperature. Thus, an organic group-terminated polyalkylene glycol-containing compound (8-2) was obtained.

<Example 6-2>

A four-necked glass recovery flask reaction vessel equipped with a thermometer, a stirrer, and a nitrogen inlet tube was charged with 20.18 g (20 mmol) of the polyalkylene glycol-containing compound (3) and 0.52 g of sodium hydroxide as a catalyst. The reaction vessel was purged with nitrogen under stirring, and the contents were heated to 120°C in a nitrogen atmosphere. The temperature was maintained for one hour under atmospheric pressure. Thereafter, the temperature was lowered to 80°C, 5.12 g (20 mmol) of lauryl glycidyl ether was added to the reaction vessel in one portion, and then the temperature was maintained for seven hours in a nitrogen atmosphere. Thereafter, the reaction solution was cooled to room temperature. Thus, an organic group-terminated polyalkylene glycol-containing compound (9-2) was obtained.

<Example 7-2>

A four-necked glass recovery flask reaction vessel equipped with a thermometer, a stirrer, and a nitrogen inlet tube was charged with 12.92 g (100 mmol) of 1-(2-hydroxyethyl)-2-pyrrolidone (HEP) and 2 g of sodium hydroxide as a catalyst. The reaction vessel was purged with nitrogen under stirring, and the contents were heated to 120°C in a nitrogen atmosphere. The temperature was maintained for one hour under atmospheric pressure. Thereafter, the temperature was lowered to 80°C, 90.3 g (100 mmol) of Denacol EX-171 (available from NCX, lauryl alcohol-EO(15)-glycidyl ether) was added to the reaction vessel in one portion, and then the temperature was maintained for seven hours in a nitrogen atmosphere. Thereafter, the reaction solution was cooled to room temperature. Thus, an organic group-terminated polyalkylene glycol-containing compound (10-2) was obtained.

<Example 8-2>

A four-necked glass recovery flask reaction vessel equipped with a thermometer, a stirrer, and a nitrogen inlet tube was charged with 12.92 g (100 mmol) of 1-(2-hydroxyethyl)-2-pyrrolidone (HEP) and 2 g of sodium hydroxide as a catalyst. The reaction vessel was purged with nitrogen under stirring, and the contents were heated to 120°C in a nitrogen atmosphere. The temperature was maintained for one hour under atmospheric pressure. Thereafter, the temperature was lowered to 80°C, 180.6 g (200 mmol) of Denacol EX-171 (available from NCX, lauryl alcohol-EO(15)-glycidyl ether) was added to the reaction vessel in one portion, and then the temperature was maintained for 20 hours in a nitrogen atmosphere. Thereafter, the reaction solution was cooled to room temperature. Thus, an organic group-terminated polyalkylene glycol-containing compound (11-2) was obtained.

<Synthesis example of monomer (1)>

A 500-ml four-necked glass recovery flask reaction vessel equipped with a thermometer, a stirrer, and a reflux tube was charged with 103.66 g (0.90 mol) of 1-(2-hydroxyethyl)-2-pyrrolidone (hereinafter, also referred to as HEP) and 249.80 g (2.70 mol) of epichlorohydrin (hereinafter, also referred to as ECH). The contents were heated to 40°C under stirring using a stirrer. Thereafter, while the temperature was maintained at 50°C or lower, 36.0 g (0.90 mol) of granular sodium hydroxide was added in ten portions. Then, 36.0 g (0.90 mol) of granular sodium hydroxide was added in two portions, followed by stirring for one hour at 50°C. Thereafter, the reaction solution was cooled to room temperature, and the produced salts were removed by suction filtration to give a coarse product. Subsequently, the coarse product was subjected to evaporation to mostly remove epichlorohydrin remaining therein and then, subjected to reduced pressure distillation to completely remove the epichlorohydrin. Thus, a pyrrolidone compound containing an epoxy ring (hereinafter, also referred to as a monomer (1) or HEP-ECH (ECH-HEP)) was obtained.

<Synthesis example of monomer (2)>

A 2-L four-necked glass recovery flask reaction vessel equipped with a thermometer, a stirrer, and a reflux tube was charged with 297.89 g (3.50 mol) of 2-pyrrolidone (hereinafter, also referred to as 2Py) and 971.46 g (10.50 mol) of epichlorohydrin. The contents were heated to 45°C under stirring using a stirrer. Thereafter, while the temperature was maintained at 50°C or lower, 140.0 g (3.50 mol) of granular sodium hydroxide was added in ten portions. Then, 21.0 g (0.53 mol) of granular sodium hydroxide was added in two portions, followed by stirring for one hour at 50°C. Thereafter, the reaction solution was cooled to room temperature, and the produced salts were removed by suction filtration to give a coarse product. Subsequently, the coarse product was subjected to evaporation to mostly remove epichlorohydrin remaining therein and then, subjected to reduced pressure distillation to completely remove the epichlorohydrin. Thus, a pyrrolidone compound containing an epoxy ring (hereinafter, also referred to as a monomer (2) or 2Py-ECH(ECH-2Py)) was obtained.

<Example 9>

A four-necked glass recovery flask reaction vessel equipped with a thermometer, a stirrer, and a nitrogen inlet tube was charged with 19.02 g (30 mmol) of NEWCOL 2310 (available from Nippon Nyukazai Co., Ltd., a compound in which an average of 10 mol of ethylene oxide was added to a C12-C13 primary alcohol, hereinafter, also referred to as NC2310) and 0.17 g (0.30 mmol) of potassium hydroxide as a catalyst. The reaction vessel was purged with nitrogen under stirring, and the contents were heated to 155°C in a nitrogen atmosphere. The temperature was maintained for one hour under atmospheric pressure. Thereafter, while 5.56 g (30 mmol) of the monomer (1) was added to the reaction vessel with a syringe at a rate of 0.1 mL/min, the temperature was maintained for four hours in a nitrogen atmosphere. Thereafter, the reaction solution was cooled to room temperature. Thus, an organic group-terminated polyalkylene glycol-containing compound (12) was obtained.

<Example 10>

A four-necked glass recovery flask reaction vessel equipped with a thermometer, a stirrer, and a nitrogen inlet tube was charged with 19.07 g (32 mmol) of SOFTANOL 90 (available from Nippon Shokubai Co., Ltd., a compound in which an average of 9 mol of ethylene oxide was added to a C12-C14 secondary alcohol, hereinafter, also referred to as SFT90) and 0.18 g (0.32 mmol) of potassium hydroxide as a catalyst. The reaction vessel was purged with nitrogen under stirring, and the contents were heated to 155°C in a nitrogen atmosphere. The temperature was maintained for one hour under atmospheric pressure. Thereafter, while 5.56 g (30 mmol) of the monomer (1) was added to the reaction vessel with a syringe at a rate of 0.1 mL/min, the temperature was maintained for four hours in a nitrogen atmosphere.

Thereafter, the reaction solution was cooled to room temperature. Thus, an organic group-terminated polyalkylene glycol-containing compound (13) was obtained.

<Example 11>

A four-necked glass recovery flask reaction vessel equipped with a thermometer, a stirrer, and a nitrogen inlet tube was charged with 20.28 g (32 mmol) of NEWCOL 2310 (available from Nippon Nyukazai Co., Ltd., a compound in which an average of 10 mol of ethylene oxide was added to a C12-C13 primary alcohol, hereinafter, also referred to as NC2310) and 0.18 g (0.32 mmol) of potassium hydroxide as a catalyst. The reaction vessel was purged with nitrogen under stirring, and the contents were heated to 155°C in a nitrogen atmosphere. The temperature was maintained for one hour under atmospheric pressure. Thereafter, while 4.52 g (32 mmol) of the monomer (2) was added to the reaction vessel with a syringe at a rate of 0.1 mL/min, the temperature was maintained for four hours in a nitrogen atmosphere. Thereafter, the reaction solution was cooled to room temperature. Thus, an organic group-terminated polyalkylene glycol-containing compound (14) was obtained.

<Example 12>

A glass reaction vessel equipped with a water quantitative receiver including a Dimroth condenser, a stirrer including a stirring blade and a stirring seal, and a temperature sensor including a glass protection tube was charged with 20.75 g of cyclohexane as an azeotropic solvent, 200 g (0.32 mol) of an adduct of lauryl alcohol with 10 mol of EO, 33.9 g (0.32 mol) of mercaptopropionic acid, 4.68 g (24.6 mmol) of paratoluenesulfonic acid monohydrate, and 0.12 g (0.59 mmol) of phenothiazine. The water quantitative receiver was filled with cyclohexane, and then the reaction system was heated to reflux under stirring. Cyclohexane was added to control the temperature of the reaction system to 110°C ± 5°C, and stirring was performed for 10 hours. Thereafter, the reaction solution was cooled to about 70°C while stirred so as not to be solidified. Then, 3.12 g (23.4 mmol) of a 30% aqueous solution of sodium hydroxide was promptly put into the reaction vessel. Next, 220 g of ion exchange water was added, and the reaction system was heated to reflux under stirring. While cyclohexane collected in the water quantitative receiver was discharged, it was completely removed from the reaction system. Thus, a thiol group-terminated polyalkylene glycol-containing compound (15) was obtained. Separately, a 500-mL glass separable flask equipped with a thermometer, a stirrer, and a reflux condenser was charged with 92.6 g of pure water. The contents were bubbled with nitrogen for 10 minutes under stirring and then heated to 80°C. Subsequently, to the separable flask were added dropwise 20.0 g (0.18 mol) of N-vinylpyrrolidone (hereinafter, also referred to as NVP), 1.95 g (0.36 mmol) of a 5% aqueous solution of 2,2'-azobis(2-methylpropioamidine)dihydrochloride (hereinafter, also referred to as V-50), 257.2 g (0.18 mol) of a 50% aqueous solution of the thiol group-terminated polyalkylene glycol-containing compound (15) through respective dropping ports. The addition times were 160 minutes for NVP, 190 minutes for V-50, and 160 minutes for the polyalkylene glycol-containing compound (15). The temperature was maintained at 80°C until the end of dropwise addition. The same temperature was further maintained for 60 minutes after the completion of the dropwise addition so that aging was performed to complete the polymerization. After the completion of the polymerization, the reaction solution was cooled to room temperature. Thus, an organic group-terminated polyalkylene glycol-containing compound (16) was obtained.

<Comparative Example 1>

A polyalkylene glycol-containing compound (1) in which an average of 10 mol of ethylene oxide was added to HEP was used.

<Comparative Example 2>

A polyalkylene glycol-containing compound (3) in which an average of 20.4 mol of ethylene oxide was added to HEP was used.

With respect to the structures of the polyalkylene glycol-containing compounds (grafted polymers) obtained in Examples 1 to 12 and Comparative Examples 1 and 2, $R^0$, $R^1$, $R^2$, $R^a$, $R^b$, $R^c$, and n in the compounds represented by the formulas (1-1), (2-1), and (3-1) are shown in Table 1 or 2. These polyalkylene glycol-containing compounds were evaluated by the above methods. The results are shown in Table 3.

[Table 1]

| | Polyalkylene glycol-containing compound | Formula | $R^0$ | $R^1$ | $R^2$ | $R^a$ | $R^b$ | $R^c$ | n |
|---|---|---|---|---|---|---|---|---|---|
| Example 1 | (4) | (1-1) | $C_2H_5$ | $C_2H_5$ | - | $C_{12}H_{25}$ | - | - | 10 |
| Example 2 | (5) | (1-1) | $C_2H_5$ | $C_2H_5$ | - | $C_{12}H_{25}$ | - | - | 20 |
| Example 3 | (6-1) or (6-2) | (1-1) | $C_2H_5$ | $C_2H_5$ | - | $CH_2CH(OH)CH_2OC_{12}H_{25}$ | - | - | 10 |
| Example 4 | (7-1) or (7-2) | (1-1) | $C_2H_5$ | $C_2H_5$ | - | $CH_2CH(OH)CH_2OC_8H_{17}$ | - | - | 10 |
| Example 5 | (8-1) or (8-2) | (2-1) | $C_2H_5$ | $C_2H_5$ | - | - | $CH_2CH(OH)CH_2OC_{18}H_{37}$ | - | 10 |
| Example 6 | (9-1) or (9-2) | (1-1) | $C_2H_5$ | $C_2H_5$ | - | $CH_2CH(OH)CH_2OC_{12}H_{25}$ | - | - | 20 |
| Example 7 | (10-1) or (10-2) | (1-1) | $CH_2CH_2OCH_2CH(OH)CH_2$ | $C_2H_5$ | - | $C_{12}H_{25}$ | - | - | 15 |
| Example 8 | (11-1) or (11-2) | (3-1) | - | $C_2H_5$ | $CH_2CH_2OCH_2CH(OCH_2CH(OH)(OCH_2CH_2)_{15}OC_{12}H_{25})CH_2$ | - | - | $C_{12}H_{25}$ | 15 |
| Comparative Examole 1 | (1) | (1-1) | $C_2H_5$ | $C_2H_5$ | - | H | - | - | 10 |
| Comparative Example 2 | (3) | (1-1) | $C_2H_5$ | $C_2H_5$ | - | H | - | - | 20 |

[Table 2]

| | Polyalkylene glycol-containing compound | Formula | $R^0$ | $R^1$ | $R^a$ | n |
|---|---|---|---|---|---|---|
| Example 9 | (12) | (1-1) | $CH_2CH_2OCH_2CH_2(OH)CH_2$ | $C_2H_5$ | C12-C13 alkyl group | 10 |
| Example 10 | (13) | (1-1) | $CH_2CH_2OCH_2CH_2(OH)CH_2$ | $C_2H_5$ | Branched C12-C14 alkyl group | 9 |
| Example 11 | (14) | (1-1) | $CH_2CH(OH)CH_2$ | $C_2H_5$ | C12-C13 alkyl group | 10 |
| Example 12 | (16) | (1-1) | $CH_2(CH_2 or CH)CH_2SCH_2CH_2CO$ | $C_2H_5$ | C12-C13 alkyl group | 10 |

[Table 3]

| | Polyalkylene glycol-containing compound | Mixed stain detergency (Without polymer: 9.5) |
|---|---|---|
| Example 1 | (4) | 24.7 |
| Example 2 | (5) | 23.8 |
| Example 3-2 | (6-2) | 25.4 |
| Example 4-2 | (7-2) | 22.3 |
| Example 5-2 | (8-2) | 21.1 |
| Example 6-2 | (9-2) | 26.3 |
| Example 7-2 | (10-2) | 24.9 |
| Example 8-2 | (11-2) | 25.3 |
| Example 9 | (12) | 26.8 |
| Example 10 | (13) | 27.1 |
| Example 11 | (14) | 26.7 |
| Example 12 | (16) | 20.9 |
| Comparative Example 1 | (1) | 13.3 |
| Comparative Example 2 | (3) | 16.3 |

## Claims

**1.** A polyalkylene glycol-containing compound represented by the following formula (1-1):

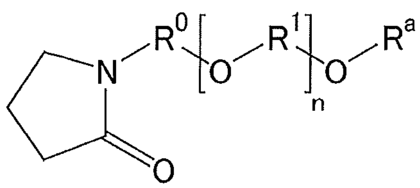

(1-1)

wherein $R^0$ is a C1-C20 divalent organic group; $R^1$s are the same as or different from each other and are each a C2-C20 alkylene group; $R^a$ is an optionally substituted C1-C20 alkyl group, an optionally substituted C6-C20 aryl group,
or an optionally substituted C1-C20 heterocyclic group; and n is an average number of moles of oxyalkylene groups added and is 1 to 300.

**2.** The polyalkylene glycol-containing compound according to claim 1, wherein $R^a$ is a C1-C20 alkyl group or a group represented by the following formula (1-2):

$$-CH_2CH(OR^{a2})CH_2-OR^{a1} \qquad (1\text{-}2)$$

wherein $R^{a1}$ is a C1-C17 alkyl or heterocyclic group or a C6-C17 aryl group; and $R^{a2}$ is a hydrogen atom, an optionally substituted C1-C16 alkyl group, an optionally substituted C6-C16 aryl group, or an optionally substituted C1-C16 heterocyclic group.

**3.** A polyalkylene glycol-containing compound represented by the following formula (2-1):

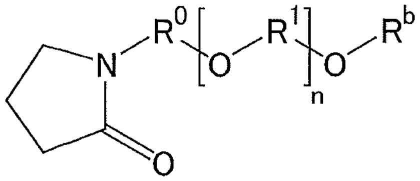

(2-1)

wherein $R^0$ is a C1-C20 divalent organic group; $R^1$s are the same as or different from each other and are each a C2-C20 alkylene group; $R^b$ is a C21-C50 organic group; and n is an average number of moles of oxyalkylene groups added and is 1 to 300.

**4.** The polyalkylene glycol-containing compound according to claim 3, wherein $R^b$ is a group represented by the following formula (2-2):

$$-CH_2CH(OR^{b2})CH_2OR^{b1} \qquad (2\text{-}2)$$

wherein $R^{b1}$ is a C1-C47 organic group; and $R^{b2}$ is a hydrogen atom or a C1-C46 organic group.

**5.** The polyalkylene glycol-containing compound according to claim 3 or 4, wherein $R^b$ is an optionally substituted C21-C30 alkyl group or an optionally substituted C21-C30 aryl group.

**6.** The polyalkylene glycol-containing compound according to any one of claims 1 to 5, wherein $R^0$ is a C1-C20 alkylene group; a group represented by the following formula (1-4):

$$-(CH_2)_p-O-CH_2CH(OR^{01})CH_2- \qquad (1\text{-}4)$$

wherein $R^{01}$ is a hydrogen atom or a C1-C17 organic group and p is 0 to 10; a group represented by the following formula (1-7):

$$-(CH_2)_{p'}-CH(OH)CH_2- \qquad (1\text{-}7)$$

wherein p' is 0 to 5; or a group represented by the following formula (1-8):

$$-R^{03}-S-R^{04}- \qquad (1\text{-}8)$$

wherein $R^{03}$ and $R^{04}$ are the same as or different from each other and are each an optionally substituted C1-C10 alkylene group.

**7.** A polyalkylene glycol-containing compound represented by the following formula (3-1):

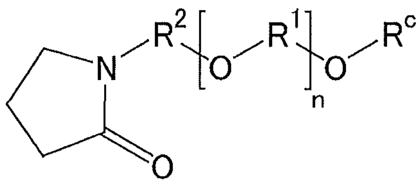

(3-1)

wherein $R^2$ is a C21-C100 divalent organic group; $R^1$s are the same as or different from each other and are each a C2-C20 alkylene group; $R^c$ is an optionally substituted C1-C50 alkyl group, an optionally substituted C6-C50 aryl group, or an optionally substituted C1-C50 heterocyclic group; and n is an average number of moles of oxyalkylene groups added and is 1 to 300.

**8.** The polyalkylene glycol-containing compound according to claim 7, wherein $R^2$ is a group represented by the following formula (3-5):

$$-CH_2CH_2OCH_2CH(OR^{22})CH_2- \qquad (3\text{-}5)$$

wherein $R^{22}$ is a C16-C95 organic group.

**9.** A composition comprising:

the polyalkylene glycol-containing compound according to any one of claims 1 to 8; and
a different component other than the compound,
the polyalkylene glycol-containing compound being present in an amount of 1 to 99% by mass based on 100% by mass of the composition.

**10.** A detergent composition comprising:

the polyalkylene glycol-containing compound according to any one of claims 1 to 8; and
a detergent additive other than the compound.

**INTERNATIONAL SEARCH REPORT**

International application No.
PCT/JP2019/002321

A. CLASSIFICATION OF SUBJECT MATTER
Int.Cl. C08G65/329(2006.01)i, C08G65/26(2006.01)i, C11D1/72(2006.01)i

According to International Patent Classification (IPC) or to both national classification and IPC

B. FIELDS SEARCHED

Minimum documentation searched (classification system followed by classification symbols)
Int.Cl. C08G65/00-67/04, C11D1/72

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | US 2015/0344461 A1 (ISP INVESTMENTS INC.) 03 December 2015, claims 1-2, paragraphs [0052]-[0057], [0205], [0305]-[0306], table 2, example 35 & WO 2014/116560 A1 | 1,9-10<br>2-8 |
| A | JP 2012-149186 A (NIPPON SHOKUBAI CO., LTD.) 09 August 2012, entire text (Family: none) | 1-10 |
| A | JP 2014-091754 A (NIPPON SHOKUBAI CO., LTD.) 19 May 2014, entire text (Family: none) | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 April 2019 (02.04.2019) | 16 April 2019 (16.04.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/002321

C (Continuation). DOCUMENTS CONSIDERED TO BE RELEVANT

| Category* | Citation of document, with indication, where appropriate, of the relevant passages | Relevant to claim No. |
|---|---|---|
| A | JP 2015-209534 A (NIPPON SHOKUBAI CO., LTD.) 24 November 2015, entire text (Family: none) | 1-10 |
| A | JP 01-502435 A (GAF CORPORATION) 24 August 1989, entire text & US 4801400 A, entire text & WO 1988/006586 A1 | 1-10 |
| P, A | JP 2018-177918 A (SHIN-ETSU CHEMICAL CO., LTD.) 15 November 2018, entire text & WO 2018/190158 A1 | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012149186 A